# EUROPEAN PATENT APPLICATION

(11) **EP 0 753 525 A1**
(43) Date of publication of application: **15.01.1997**
(21) Application number: 95913391.9
(22) Date of filing: 31.03.1995
(51) Int. Cl.: C07H 19/213, A61K 31/70

(54) **CYCLIC NUCLEOTIDE DERIVATIVE**

(30) Priority: 31.03.1994 JP 63344/94
(71) Applicant: NIPPON SHINYAKU COMPANY, LIMITED, Minami-ku Kyoto-shi Kyoto 601 (JP)
(72) Inventor: OHGI, Tadaaki, Otsu-shi Shiga 520 (JP); SATO, Yoshitake, Muko-shi Kyoto 617 (JP); MAKITA, Yoshihiko, Takatsuki-shi Osaka 569 (JP); TATSUMI, Shigeru Nipponshinyaku Co., Ltd., Kyoto-shi Kyoto 607 (JP)
(74) Representative: Strych, Werner Maximilian Josef, Dr.
(86) International application number: JP9500617
(87) International publication number: WO9526971

(57) **Abstract**

The object of the present invention is to provide useful cyclic nucleotide derivatives, for example, as an antidementia drug, an antiarrhythmic drug, a therapeutic drug for bronchial asthma or an antiinflammatory agent.

The invention relates to cyclic nucleotide derivatives of the following general formula [I] and the pharmaceutically acceptable salts thereof. 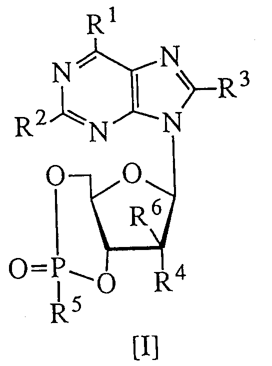 wherein R¹ represents hydroxy, amino, or -NR¹¹R¹² in which R¹¹ and R¹² may be the same or different and each represents alkyl; R² represents hydrogen, amino, or hydroxy; R³ represents hydrogen, halogen, alkyl, hydroxy or mercapto; R⁴ represents hydrogen, hydroxy, acyloxy or alkoxy; R⁵ represents alkyl or aryl; R⁶ represents hydrogen, halogen or alkyl.

## Description

### TECHNICAL FIELD

The present invention relates to useful cyclic nucleotide derivatives as a medicine.

### BACKGROUND TECHNOLOGY

Cyclic AMP and cyclic GMP are known as intracellular messengers. Phosphodiesterases (PDE), the enzymes which decompose these cyclic nucleotides, regulate the concentrations of messengers are involved in many physiologic reactions such as aggregation of platelets, relaxation of blood vessels, and dilatation of the trachea, among others.

According to recent studies, PDE is classified into the five isozymes I, II, III, IV, and V, which are dissimilar in the in vivo distribution. For example, the isozyme I is occurs in the central nervous system, liver, fat cell, heart, and kidney, while the isozyme II is distributed in the heart, adrenal cortex, central nervous system, airway smooth muscle, and kidney. The isozyme III is present in the platelet, smooth muscle, heart, fat cell, central nervous system, kidney, and liver. The isozyme IV is distributed in the inflammatory cell, smooth muscle, brain, liver, heart, and kidney. The isozyme V is known to be present in the retina, smooth muscle, and platelet. (I. P. Hall, Br. J. Clin, Pharmac., 35, p. 1-7, (1993)).

Assuming that each of these isozymes could be selectively inhibited, the level of the cyclic nucleotide in a given tissue could be increased so that various physiologic reactions such as platelet aggregation, vascular relaxation, and tracheal dilatation could be modulated. For example, by selective inhibition of the isozyme I, a metabolic improvement effect of cerebral circulation or a vascular smooth muscle relaxant effect can be expected (I. P. Hall, Br. J. Clin. Pharmac., 35, p. 1-7 (1993); Miyazaki, Manabu: Japanese Pharmacology & Therapeutics, 10, p. 1945 (1982); Tamaki, Norihiko: The Japanese Journal of Clinical and Experimental Medicine, 59, p. 4135 (1982)). An inhibitor of the isozyme II is under development as an antiarrhythmic drug (T. Prodzuweit et al., The Lancet, 341, p. 760 (1993)).

By inhibiting the isozyme III, inhibition of platelet aggregation or relaxation of vascular smooth muscle can be achieved. An inhibitor of the isozyme IV can be used as a therapeutic agent for bronchial asthma or an antiinflammatory agent and an inhibitor of the isozyme V can serve as a platelet aggregation inhibitor (I. P. Hall, Br. J. Clin. Pharmac., 35, p. 1-7 (1993), C. D. Nicolson et al., Trends Pharmac. Sci., 12, p. 19-27 (1991)).

Cyclic nucleotide derivatives as analogs of the substrates for PDEs have been most extensively studied of all kinds of PDE inhibitors and a large number of cyclic nucleotide derivatives having useful PDE-inhibitory activity or platelet aggregation-inhibitory activity have been reported until now.

Japanese Laid-Open S63-135399 discloses adenosine 3',5'-cyclic methylphosphonate, for instance.

Japanese Laid-Open H2-223590 discloses 8-bromoadenosine 3',5'-cyclic methylphosphonate, N-benzoyladenosine 3',5'-cyclic methylphosphonate, N-benzoyl-2'-O-deoxyadenosine 3',5'-cyclic methylphosphonate, guanosine 3',5'-cyclic methylphosphonate, 8-bromoguanosine 3',5'-cyclic methylphosphonate, N-n-butyryl-2'-O-n-butyryladenosine 3',5'-cyclic methylphosphonate, etc.

PCT WO91/19728 discloses inosine 3',5'-cyclic methylphosphonate, 8-bromoinosine 3',5'-cyclic methylphosphonate, etc.

However, comparatively large amounts of these derivatives are needed in order that the desired efficacy may be exhibited in the in vivo environment and have not been established to be selective for any specific PDE isozyme or, if established, are low in the selectivity.

### DISCLOSURE OF THE INVENTION

The object of the present invention is to find and thereby provide medicinally useful cyclic nucleotide derivatives differing from the above known cyclic nucleotide derivatives and characterized not only by higher PDE inhibitory activity than that of the known derivatives but also by high selectivity for respective PDE isozymes I, II, and IV.

To accomplish the above object, the inventors of the present invention synthesized varieties of cyclic AMP derivatives and cyclic GMP derivatives and investigated their pharmacologic profiles. In the course of the investigation, the inventors discovered compounds meeting the above object and have perfected the present invention.

The compound of the present invention is a novel compound not heretofore described in any literature, which has the following formula [I]. 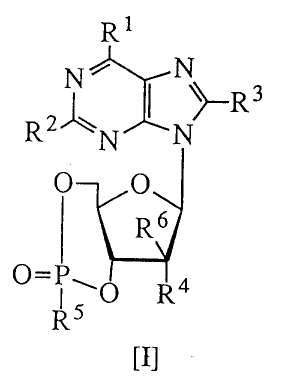 wherein
R¹ represents hydroxy, amino, or -NR¹¹R¹² in which R¹¹ and R¹² may be the same or different and each represents alkyl.
R² represents hydrogen, amino, or hydroxy.
R³ represents hydrogen, halogen, alkyl, hydroxy, mercapto, or -S-X where X represents alkyl which is either unsubstituted or substituted (the substituent is selected from among hydroxy, carboxy and alkoxyalkoxy), cycloalkyl, or aryl which is either unsubstituted or substituted by halogen.
R⁴ represents hydrogen, hydroxy, acyloxy, alkoxy, or -OSO₂-Y wherein Y represents aryl which is either unsubstituted or substituted by alkyl or alkyl which is either unsubstituted or substituted by halogen.
R⁵ represents alkyl or aryl.
R⁶ represents hydrogen, halogen, or alkyl.
R⁴ and R⁶ may jointly represent =CH₂.
R³ and R⁶ may jointly represent -S-.

In the case that R⁴ and R⁶ are independent of each other, at least either one represents hydrogen.

Excluded are the case in which R¹ represents amino, R² represents hydrogen, R³ represents hydrogen or halogen, R⁴ represents hydrogen, hydroxy, or acyloxy, R⁵ represents alkyl, and R⁶ represents hydrogen, the case in which R¹ represents hydroxy, R² represents hydrogen, R³ represents hydrogen, halogen, alkyl, or hydroxy, R⁴ represents hydrogen, hydroxy, acyloxy, or alkoxy, R⁵ represents alkyl, and R⁶ represents hydrogen, and the case in which R¹ represents hydroxy, R² represents amino, R³ represents hydrogen or halogen, R⁴ represents hydrogen, hydroxy, or acyloxy, R⁵ represents alkyl, and R⁶ represents hydrogen.

Referring to formula [I], the alkyl for R¹¹ and R¹² is preferably a straight-chain or branched-chain alkyl group of 1-7 carbon atoms, thus including methyl, ethyl, n-propyl, isopropyl, n-butyl, isobutyl, tert-butyl, n-pentyl, isopentyl, n-hexyl, isohexyl, n-heptyl, isoheptyl, etc.

The halogen for R³ may be fluorine, chlorine, bromine, or iodine.

The alkyl for R³ includes the alkyl groups mentioned above as examples of R¹¹ and R¹².

The alkyl for X includes the same alkyl groups as mentioned above for R¹¹ and R¹². The alkoxyalkoxy mentioned above as a substituent for said alkyl is preferably a straight-chain or branched-chain C₁₋₇ alkoxy C₁₋₇ alkoxy group. Here, the alkyl moiety of each alkoxy includes the same alkyl groups as mentioned above for R¹¹ and R¹².

The cycloalkyl for X is preferably a group of 3-7 carbon atoms, thus including cyclopropyl, cyclobutyl, cyclopentyl, cyclohexyl, and cycloheptyl, among others.

The aryl for X is preferably an aryl group of 6-10 carbon atoms, such as phenyl and naphthyl. The halogen as a substituent for said aryl may be fluorine, chlorine, bromine, or iodine.

The acyloxy for R⁴ is preferably a straight-chain or branched-chain acyloxy group containing 1-7 carbon atoms, thus including formyloxy, acetyloxy, propionyloxy, butyryloxy, isobutyryloxy, valeryloxy, isovaleryloxy, pivaloyloxy, hexanoyloxy, isohexanoyloxy, heptanoyloxy, and isoheptanoyloxy, among others.

The alkyl moiety of the alkoxy mentioned for R⁴ includes the same alkyl groups as mentioned above for R¹¹ and R¹².

The aryl for Y includes the same aryl groups as mentioned above as examples of X. The alkyl mentioned as a substituent for said aryl includes the same alkyl groups as mentioned above for R¹¹ and R¹².

The alkyl for Y includes the same alkyl groups as mentioned for R¹¹ R¹². The halogen mentioned as a substituent for said alkyl may be fluorine, chlorine, bromine, or iodine.

The alkyl for R⁵ includes the same alkyl groups as mentioned for R¹¹ and R¹².

The aryl for R⁵ includes the same aryl groups as mentioned for X.

The halogen for R⁶ may be fluorine, chlorine, bromine, or iodine.

The alkyl for R⁶ includes the same alkyl groups as mentioned for R¹¹ and R¹².

The pharmaceutically acceptable salt includes salts with mineral acids such as hydrochloric acid, hydrobromic acid, sulfuric acid, phosphoric acid, etc. and salts with organic acids such as acetic acid, citric acid, tartaric acid, maleic acid, succinic acid, fumaric acid, p-toluenesulfonic acid, benzenesulfonic acid, methanesulfonic acid, etc.

The compound of the present invention includes not only the compounds mentioned specifically in the production examples which appear hereinafter but also the compounds listed hereinafter. It should be understood that these compounds are mentioned only as typical example and does not limit the scope of the invent.

It should also be understood that because of the asymmetric phosphorus atom contained, the compound of the present invention may exist in two optically active forms and that each of these optically active compounds and mixtures of them also fall within the scope of the invention.
2'-O-acetyl-8-mercaptoadenosine 3',5'-cyclic methylphosphonate,
2'-O-propionyl-8-mercaptoadenosine 3',5'-cyclic methylphosphonate,
2'-O-pivaloyl-8-mercaptoadenosine 3',5'-cyclic methylphosphonate,
2'-O-acetyl-8-methylthioadenosine 3',5'-cyclic methylphosphonate,
2'-O-propionyl-8-methylthioadenosine 3',5'-cyclic methylphosphonate,
2'-O-pivaloyl-8-methylthioadenosine 3',5'-cyclic methylphosphonate,
2'-O-acetyl-8-ethylthioadenosine 3',5'-cyclic methylphosphonate,
2'-O-propionyl-8-ethylthioadenosine 3',5'-cyclic methylphosphonate,
2'-O-pivaloyl-8-ethylthioadenosine 3',5'-cyclic methylphosphonate,
2'-O-acetyl-8-propylthioadenosine 3',5'-cyclic methylphosphonate,
2'-O-propionyl-8-propylthioadenosine 3',5'-cyclic methylphosphonate,
2'-O-pivaloyl-8-propylthioadenosine 3',5'-cyclic methylphosphonate,
2'-O-acetyl-8-isopropylthioadenosine 3',5'-cyclic methylphosphonate,
2'-O-propionyl-8-isopropylthioadenosine 3',5'-cyclic methylphosphonate,
2'-O-pivaloyl-8-isopropylthioadenosine 3',5'-cyclic methylphosphonate,
2'-O-acetyl-8-cyclopentylthioadenosine 3',5'-cyclic methylphosphonate,
2'-O-propionyl-8-cyclopentylthioadenosine 3',5'-cyclic methylphosphonate,
2'-O-pivaloyl-8-cyclopentylthioadenosine 3',5'-cyclic methylphosphonate,
2'-O-acetyl-8-(2-hydroxyethylthio)adenosine 3',5'-cyclic methylphosphonate,
2'-O-propionyl-8-(2-hydroxyethylthio)adenosine 3',5'-cyclic methylphosphonate,
2'-O-pivaloyl-8-(2-hydroxyethylthio)adenosine 3',5'-cyclic methylphosphonate,
2'-O-acetyl-8-(3-hydroxypropylthio)adenosine 3',5'-cyclic methylphosphonate,
2'-O-propionyl-8-(3-hydroxypropylthio)adenosine 3',5'-cyclic methylphosphonate,
2'-O-pivaloyl-8-(3-hydroxypropylthio)adenosine 3',5'-cyclic methylphosphonate,
2'-O-acetyl-8-[2-(2-methoxyethoxy)ethylthio]adenosine 3',5'-cyclic methylphosphonate,
2'-O-propionyl-8-[2-(2-methoxyethoxy)ethylthio]adenosine 3',5'-cyclic methylphosphonate,
2'-O-pivaloyl-8-[2-(2-methoxyethoxy)ethylthio]adenosine 3',5'-cyclic methylphosphonate,
2'-O-acetyl-8-carboxymethylthioadenosine 3',5'-cyclic methylphosphonate,
2'-O-propionyl-8-carboxymethylthioadenosine 3',5'-cyclic methylphosphonate,
2'-O-pivaloyl-8-carboxymethylthioadenosine 3',5'-cyclic methylphosphonate,
2'-O-pivaloyl-8-mercaptoguanosine 3',5'-cyclic methylphosphonate,
2'-O-acetyl-8-methylthioguanosine 3',5'-cyclic methylphosphonate,
2'-O-propionyl-8-methylthioguanosine 3',5'-cyclic methylphosphonate,
2'-O-pivaloyl-8-methylthioguanosine 3',5'-cyclic methylphosphonate,
2'-O-acetyl-8-ethylthioguanosine 3',5'-cyclic methylphosphonate,
2'-O-propionyl-8-ethylthioguanosine 3',5'-cyclic methylphosphonate,
2'-O-pivaloyl-8-ethylthioguanosine 3',5'-cyclic methylphosphonate,
2'-O-acetyl-8-propylthioguanosine 3',5'-cyclic methylphosphonate,
2'-O-propionyl-8-propylthioguanosine 3',5'-cyclic methylphosphonate,
2'-O-pivaloyl-8-propylthioguanosine 3',5'-cyclic methylphosphonate,
2'-O-acetyl-8-isopropylthioguanosine 3',5'-cyclic methylphosphonate,
2'-O-propionyl-8-isopropylthioguanosine 3',5'-cyclic methylphosphonate,
2'-O-pivaloyl-8-isopropylthioguanosine 3',5'-cyclic methylphosphonate,
2'-O-acetyl-8-cyclopentylthioguanosine 3',5'-cyclic methylphosphonate,
2'-O-propionyl-8-cyclopentylthioguanosine 3',5'-cyclic methylphosphonate,
2'-O-pivaloyl-8-cyclopentylthioguanosine 3',5'-cyclic methylphosphonate,
2'-O-pivaloyl-8-(2-hydroxyethylthio)guanosine 3',5'-cyclic methylphosphonate,
2'-O-pivaloyl-8-(3-hydroxypropylthio)guanosine 3',5'-cyclic methylphosphonate,
2'-O-acetyl-8-[2-(2-methoxyethoxy)ethylthio]guanosine 3',5'-cyclic methylphosphonate,
2'-O-propionyl-8-[2-(2-methoxyethoxy)ethylthio]guanosine 3',5'-cyclic methylphosphonate,
2'-O-pivaloyl-8-(2-(2-methoxyethoxy)ethylthio]guanosine 3',5'-cyclic methylphosphonate,
2'-O-acetyl-8-carboxymethylthioguanosine 3',5'-cyclic methylphosphonate,
2'-O-propionyl-8-carboxymethylthioguanosine 3',5'-cyclic methylphosphonate,
2'-O-pivaloyl-8-carboxymethylthioguanosine 3',5'-cyclic methylphosphonate,
2'-O-methyladenosine 3',5'-cyclic methylphosphonate,
2'-O-methyladenosine 3',5'-cyclic ethylphosphonate,
2'-O-methyladenosine 3',5'-cyclic propylphosphonate,
2'-O-ethyladenosine 3',5'-cyclic methylphosphonate,
2'-O-ethyladenosine 3',5'-cyclic ethylphosphonate,
2'-O-ethyladenosine 3',5'-cyclic propylphosphonate,
2'-O-propyladenosine 3',5'-cyclic methylphosphonate,
2'-O-propyladenosine 3',5'-cyclic ethylphosphonate,
2'-O-propyladenosine 3',5'-cyclic propylphosphonate,
2'-O-methyl-8-bromoadenosine 3',5'-cyclic methylphosphonate,
2'-O-methyl-8-bromoadenosine 3',5'-cyclic ethylphosphonate,
2'-O-methyl-8-bromoadenosine 3',5'-cyclic propylphosphonate,
2'-O-ethyl-8-bromoadenosine 3',5'-cyclic methylphosphonate,
2'-O-ethyl-8-bromoadenosine 3',5'-cyclic ethylphosphonate,
2'-O-ethyl-8-bromoadenosine 3',5'-cyclic propylphosphonate,
2'-O-propyl-8-bromoadenosine 3',5'-cyclic methylphosphonate,
2'-O-propyl-8-bromoadenosine 3',5'-cyclic ethylphosphonate,
2'-O-propyl-8-bromoadenosine 3',5'-cyclic propylphosphonate,
2'-O-methyl-8-mercaptoadenosine 3',5'-cyclic methylphosphonate,
2'-O-methyl-8-mercaptoadenosine 3',5'-cyclic ethylphosphonate,
2'-O-methyl-8-mercaptoadenosine 3',5'-cyclic propylphosphonate,
2'-O-ethyl-8-mercaptoadenosine 3',5'-cyclic methylphosphonate,
2'-O-ethyl-8-mercaptoadenosine 3',5'-cyclic ethylphosphonate,
2'-O-ethyl-8-mercaptoadenosine 3',5'-cyclic propylphosphonate,
2'-O-propyl-8-mercaptoadenosine 3',5'-cyclic methylphosphonate,
2'-O-propyl-8-mercaptoadenosine 3',5'-cyclic ethylphosphonate,
2'-O-propyl-8-mercaptoadenosine 3',5'-cyclic propylphosphonate,
2'-O-methyl-8-methylthioadenosine 3',5'-cyclic methylphosphonate,
2'-O-methyl-8-methylthioadenosine 3',5'-cyclic ethylphosphonate,
2'-O-methyl-8-methylthioadenosine 3',5'-cyclic propylphosphonate,
2'-O-ethyl-8-methylthioadenosine 3',5'-cyclic methylphosphonate,
2'-O-ethyl-8-methylthioadenosine 3',5'-cyclic ethylphosphonate,
2'-O-ethyl-8-methylthioadenosine 3',5'-cyclic propylphosphonate,
2'-O-propyl-8-methylthioadenosine 3',5'-cyclic methylphosphonate,
2'-O-propyl-8-methylthioadenosine 3',5'-cyclic ethylphosphonate,
2'-O-propyl-8-methylthioadenosine 3',5'-cyclic propylphosphonate,
2'-O-methyl-8-ethylthioadenosine 3',5'-cyclic methylphosphonate,
2'-O-methyl-8-ethylthioadenosine 3',5'-cyclic ethylphosphonate,
2'-O-methyl-8-ethylthioadenosine 3',5'-cyclic propylphosphonate,
2'-O-ethyl-8-ethylthioadenosine 3',5'-cyclic methylphosphonate,
2'-O-ethyl-8-ethylthioadenosine 3',5'-cyclic ethylphosphonate,
2'-O-ethyl-8-ethylthioadenosine 3',5'-cyclic propylphosphonate,
2'-O-propyl-8-ethylthioadenosine 3',5'-cyclic methylphosphonate,
2'-O-propyl-8-ethylthioadenosine 3',5'-cyclic ethylphosphonate,
2'-O-propyl-8-ethylthioadenosine 3',5'-cyclic propylphosphonate,
2'-O-methyl-8-propylthioadenosine 3',5'-cyclic methylphosphonate,
2'-O-methyl-8-propylthioadenosine 3',5'-cyclic ethylphosphonate,
2'-O-methyl-8-propylthioadenosine 3',5'-cyclic propylphosphonate,
2'-O-ethyl-8-propylthioadenosine 3',5'-cyclic methylphosphonate,
2'-O-ethyl-8-propylthioadenosine 3',5'-cyclic ethylphosphonate,
2'-O-ethyl-8-propylthioadenosine 3',5'-cyclic propylphosphonate,
2'-O-propyl-8-propylthioadenosine 3',5'-cyclic methylphosphonate,
2'-O-propyl-8-propylthioadenosine 3',5'-cyclic ethylphosphonate,
2'-O-propyl-8-propylthioadenosine 3',5'-cyclic propylphosphonate,
2'-O-methyl-8-isopropylthioadenosine 3',5'-cyclic methylphosphonate,
2'-O-methyl-8-isopropylthioadenosine 3',5'-cyclic ethylphosphonate,
2'-O-methyl-8-isopropylthioadenosine 3',5'-cyclic propylphosphonate,
2'-O-ethyl-8-isopropylthioadenosine 3',5'-cyclic methylphosphonate,
2'-O-ethyl-8-isopropylthioadenosine 3',5'-cyclic ethylphosphonate,
2'-O-ethyl-8-isopropylthioadenosine 3',5'-cyclic propylphosphonate,
2'-O-propyl-8-isopropylthioadenosine 3',5'-cyclic methylphosphonate,
2'-O-propyl-8-isopropylthioadenosine 3',5'-cyclic ethylphosphonate,
2'-O-propyl-8-isopropylthioadenosine 3',5'-cyclic propylphosphonate,
2'-O-methyl-8-cyclopropylthioadenosine 3',5'-cyclic methylphosphonate,
2'-O-methyl-8-cyclopropylthioadenosine 3',5'-cyclic ethylphosphonate,
2'-O-methyl-8-cyclopropylthioadenosine 3',5'-cyclic propylphosphonate,
2'-O-ethyl-8-cyclopropylthioadenosine 3',5'-cyclic methylphosphonate,
2'-O-ethyl-8-cyclopropylthioadenosine 3',5'-cyclic ethylphosphonate,
2'-O-ethyl-8-cyclopropylthioadenosine 3',5'-cyclic propylphosphonate,
2'-O-propyl-8-cyclopropylthioadenosine 3',5'-cyclic methylphosphonate,
2'-O-propyl-8-cyclopropylthioadenosine 3',5'-cyclic ethylphosphonate,
2'-O-propyl-8-cyclopropylthioadenosine 3',5'-cyclic propylphosphonate,
2'-O-methyl-8-(2-hydroxyethylthio)adenosine 3',5'-cyclic methylphosphonate,
2'-O-methyl-8-(2-hydroxyethylthio)adenosine 3',5'-cyclic ethylphosphonate,
2'-O-methyl-8-(2-hydroxyethylthio)adenosine 3',5'-cyclic propylphosphonate,
2'-O-ethyl-8-(2-hydroxyethylthio)adenosine 3',5'-cyclic methylphosphonate,
2'-O-ethyl-8-(2-hydroxyethylthio)adenosine 3',5'-cyclic ethylphosphonate,
2'-O-ethyl-8-(2-hydroxyethylthio)adenosine 3',5'-cyclic propylphosphonate,
2'-O-propyl-8-(2-hydroxyethylthio)adenosine 3',5'-cyclic methylphosphonate,
2'-O-propyl-8-(2-hydroxyethylthio)adenosine 3',5'-cyclic ethylphosphonate,
2'-O-propyl-8-(2-hydroxyethylthio)adenosine 3',5'-cyclic propylphosphonate,
2'-O-methyl-8-[2-(2-methoxyethoxy)ethylthio]adenosine 3',5'-cyclic methylphosphonate,
2'-O-methyl-8-[2-(2-methoxyethoxy)ethylthio]adenosine 3',5'-cyclic ethylphosphonate,
2'-O-methyl-8-[2-(2-methoxyethoxy)ethylthio]adenosine 3',5'-cyclic propylphosphonate,
2'-O-ethyl-8-[2-(2-methoxyethoxy)ethylthio]adenosine 3',5'-cyclic methylphosphonate,
2'-O-ethyl-8-[2-(2-methoxyethoxy)ethylthio]adenosine 3',5'-cyclic ethylphosphonate,
2'-O-ethyl-8-[2-(2-methoxyethoxy)ethylthio]adenosine 3',5'-cyclic propylphosphonate,
2'-O-propyl-8-[2-(2-methoxyethoxy)ethylthio]adenosine 3',5'-cyclic methylphosphonate,
2'-O-propyl-8-[2-(2-methoxyethoxy)ethylthio]adenosine 3',5'-cyclic ethylphosphonate,
2'-O-propyl-8-[2-(2-methoxyethoxy)ethylthio]adenosine 3',5'-cyclic propylphosphonate,
2'-O-ethyl-8-(3-hydroxypropylthio)adenosine 3',5'-cyclic methylphosphonate,
2'-O-ethyl-8-(3-hydroxypropylthio)adenosine 3',5'-cyclic ethylphosphonate,
2'-O-propyl-8-(3-hydroxypropylthio)adenosine 3',5'-cyclic propylphosphonate,
2'-O-methylguanosine 3',5'-cyclic methylphosphonate,
2'-O-methylguanosine 3',5'-cyclic ethylphosphonate,
2'-O-methylguanosine 3',5'-cyclic propylphosphonate,
2'-O-ethylguanosine 3',5'-cyclic methylphosphonate,
2'-O-ethylguanosine 3',5'-cyclic ethylphosphonate,
2'-O-ethylguanosine 3',5'-cyclic propylphosphonate,
2'-O-propylguanosine 3',5'-cyclic methylphosphonate,
2'-O-propylguanosine 3',5'-cyclic ethylphosphonate,
2'-O-propylguanosine 3',5'-cyclic propylphosphonate,
2'-O-methyl-8-bromoguanosine 3',5'-cyclic methylphosphonate,
2'-O-methyl-8-bromoguanosine 3',5'-cyclic ethylphosphonate,
2'-O-methyl-8-bromoguanosine 3',5'-cyclic propylphosphonate,
2'-O-ethyl-8-bromoguanosine 3',5'-cyclic methylphosphonate,
2'-O-ethyl-8-bromoguanosine 3',5'-cyclic ethylphosphonate,
2'-O-ethyl-8-bromoguanosine 3',5'-cyclic propylphosphonate,
2'-O-propyl-8-bromoguanosine 3',5'-cyclic methylphosphonate,
2'-O-propyl-8-bromoguanosine 3',5'-cyclic ethylphosphonate,
2'-O-propyl-8-bromoguanosine 3',5'-cyclic propylphosphonate,
2'-O-methyl-8-mercaptoguanosine 3',5'-cyclic methylphosphonate,
2'-O-methyl-8-mercaptoguanosine 3',5'-cyclic ethylphosphonate,
2'-O-methyl-8-mercaptoguanosine 3',5'-cyclic propylphosphonate,
2'-O-ethyl-8-mercaptoguanosine 3',5'-cyclic methylphosphonate,
2'-O-ethyl-8-mercaptoguanosine 3',5'-cyclic ethylphosphonate,
2'-O-ethyl-8-mercaptoguanosine 3',5'-cyclic propylphosphonate,
2'-O-propyl-8-mercaptoguanosine 3',5'-cyclic methylphosphonate,
2'-O-propyl-8-mercaptoguanosine 3',5'-cyclic ethylphosphonate,
2'-O-propyl-8-mercaptoguanosine 3',5'-cyclic propylphosphonate,
2'-O-methyl-8-methylthioguanosine 3',5'-cyclic methylphosphonate,
2'-O-methyl-8-methylthioguanosine 3',5'-cyclic ethylphosphonate,
2'-O-methyl-8-methylthioguanosine 3',5'-cyclic propylphosphonate,
2'-O-ethyl-8-methylthioguanosine 3',5'-cyclic methylphosphonate,
2'-O-ethyl-8-methylthioguanosine 3',5'-cyclic ethylphosphonate,
2'-O-ethyl-8-methylthioguanosine 3',5'-cyclic propylphosphonate,
2'-O-propyl-8-methylthioguanosine 3',5'-cyclic methylphosphonate,
2'-O-propyl-8-methylthioguanosine 3',5'-cyclic ethylphosphonate,
2'-O-propyl-8-methylthioguanosine 3',5'-cyclic propylphosphonate,
2'-O-methyl-8-ethylthioguanosine 3',5'-cyclic methylphosphonate,
2'-O-methyl-8-ethylthioguanosine 3',5'-cyclic ethylphosphonate,
2'-O-methyl-8-ethylthioguanosine 3',5'-cyclic propylphosphonate,
2'-O-ethyl-8-ethylthioguanosine 3',5'-cyclic methylphosphonate,
2'-O-ethyl-8-ethylthioguanosine 3',5'-cyclic ethylphosphonate,
2'-O-ethyl-8-ethylthioguanosine 3',5'-cyclic propylphosphonate,
2'-O-propyl-8-ethylthioguanosine 3',5'-cyclic methylphosphonate,
2'-O-propyl-8-ethylthioguanosine 3',5'-cyclic ethylphosphonate,
2'-O-propyl-8-ethylthioguanosine 3',5'-cyclic propylphosphonate,
2'-O-methyl-8-propylthioguanosine 3',5'-cyclic methylphosphonate,
2'-O-methyl-8-propylthioguanosine 3',5'-cyclic ethylphosphonate,
2'-O-methyl-8-propylthioguanosine 3',5'-cyclic propylphosphonate,
2'-O-ethyl-8-propylthioguanosine 3',5'-cyclic methylphosphonate,
2'-O-ethyl-8-propylthioguanosine 3',5'-cyclic ethylphosphonate,
2'-O-ethyl-8-propylthioguanosine 3',5'-cyclic propylphosphonate,
2'-O-propyl-8-propylthioguanosine 3',5'-cyclic methylphosphonate,
2'-O-propyl-8-propylthioguanosine 3',5'-cyclic ethylphosphonate,
2'-O-propyl-8-propylthioguanosine 3',5'-cyclic propylphosphonate,
2'-O-methyl-8-isopropylthioguanosine 3',5'-cyclic methylphosphonate,
2'-O-methyl-8-isopropylthioguanosine 3',5'-cyclic ethylphosphonate,
2'-O-methyl-8-isopropylthioguanosine 3',5'-cyclic propylphosphonate,
2'-O-ethyl-8-isopropylthioguanosine 3',5'-cyclic methylphosphonate,
2'-O-ethyl-8-isopropylthioguanosine 3',5'-cyclic ethylphosphonate,
2'-O-ethyl-8-isopropylthioguanosine 3',5'-cyclic propylphosphonate,
2'-O-propyl-8-isopropylthioguanosine 3',5'-cyclic methylphosphonate,
2'-O-propyl-8-isopropylthioguanosine 3',5'-cyclic ethylphosphonate,
2'-O-propyl-8-isopropylthioguanosine 3',5'-cyclic propylphosphonate,
2'-O-methyl-8-cyclopropylthioguanosine 3',5'-cyclic methylphosphonate,
2'-O-methyl-8-cyclopropylthioguanosine 3',5'-cyclic ethylphosphonate,
2'-O-methyl-8-cyclopropylthioguanosine 3',5'-cyclic propylphosphonate,
2'-O-ethyl-8-cyclopropylthioguanosine 3',5'-cyclic methylphosphonate,
2'-O-ethyl-8-cyclopropylthioguanosine 3',5'-cyclic ethylphosphonate,
2'-O-ethyl-8-cyclopropylthioguanosine 3',5'-cyclic propylphosphonate,
2'-O-propyl-8-cyclopropylthioguanosine 3',5'-cyclic methylphosphonate,
2'-O-propyl-8-cyclopropylthioguanosine 3',5'-cyclic ethylphosphonate,
2'-O-propyl-8-cyclopropylthioguanosine 3',5'-cyclic propylphosphonate,
2'-O-methyl-8-(2-hydroxyethylthio)guanosine 3',5'-cyclic methylphosphonate,
2'-O-methyl-8-(2-hydroxyethylthio)guanosine 3',5'-cyclic ethylphosphonate,
2'-O-methyl-8-(2-hydroxyethylthio)guanosine 3',5'-cyclic propylphosphonate,
2'-O-ethyl-8-(2-hydroxyethylthio)guanosine 3',5'-cyclic methylphosphonate,
2'-O-ethyl-8-(2-hydroxyethylthio)guanosine 3',5'-cyclic ethylphosphonate,
2'-O-ethyl-8-(2-hydroxyethylthio)guanosine 3',5'-cyclic propylphosphonate,
2'-O-propyl-8-(2-hydroxyethylthio)guanosine 3',5'-cyclic methylphosphonate,
2'-O-propyl-8-(2-hydroxyethylthio)guanosine 3',5'-cyclic ethylphosphonate,
2'-O-propyl-8-(2-hydroxyethylthio)guanosine 3',5'-cyclic propylphosphonate,
2'-O-methyl-8-[2-(2-methoxyethoxy)ethylthio]guanosine 3',5'-cyclic methylphosphonate,
2'-O-methyl-8-[2-(2-methoxyethoxy)ethylthio]guanosine 3',5'-cyclic ethylphosphonate,
2'-O-methyl-8-[2-(2-methoxyethoxy)ethylthio]guanosine 3',5'-cyclic propylphosphonate,
2'-O-ethyl-8-[2-(2-methoxyethoxy)ethylthio]guanosine 3',5'-cyclic methylphosphonate,
2'-O-ethyl-8-[2-(2-methoxyethoxy)ethylthio]guanosine 3',5'-cyclic ethylphosphonate,
2'-O-ethyl-8-[2-(2-methoxyethoxy)ethylthio]guanosine 3',5'-cyclic propylphosphonate,
2'-O-propyl-8-[2-(2-methoxyethoxy)ethylthio]guanosine 3',5'-cyclic methylphosphonate,
2'-O-propyl-8-[2-(2-methoxyethoxy)ethylthio]guanosine 3',5'-cyclic ethylphosphonate,
2'-O-propyl-8-[2-(2-methoxyethoxy)ethylthio]guanosine 3',5'-cyclic propylphosphonate,
2'-O-ethyl-8-(3-hydroxypropylthio)guanosine 3',5'-cyclic methylphosphonate,
2'-O-ethyl-8-(3-hydroxypropylthio)guanosine 3',5'-cyclic ethylphosphonate,
2'-O-propyl-8-(3-hydroxypropylthio)guanosine 3',5'-cyclic propylphosphonate.

Among specific compounds of general formula [I], the compounds selected according to the following criteria selectively inhibit PDE isozyme I. These compounds are hereinafter collectively referred to as Group A.

The compound in which R¹ is hydroxy, R² is amino, R³ is alkyl, hydroxy, mercapto, or preferably -S-X (in which X is unsubstituted alkyl or preferably alkyl substituted by hydroxy, carboxy, or alkoxyalkoxy), R⁴ is alkoxy or preferably hydrogen, hydroxy, or acyloxy, R⁵ is aryl, preferably aryl, and R⁶ is hydrogen and the pharmaceutically acceptable salt thereof.

Particularly preferred is the compound in which R¹ is hydroxy, R² is amino, R³ is 2-hydroxyethylthio, R⁴ is acetoxy, R⁵ is propyl, and R⁶ is hydrogen and the pharmaceutically acceptable salt thereof.

Among specific compounds of general formula [I], the compounds selected according to the following criteria selectively inhibit PDE isozyme II. These compounds are hereinafter referred to collectively as Group B.

The compound in which R¹ is amino or -NR¹¹R¹² (R¹¹ and R¹² may be the same or different and each represents alkyl), R² is hydrogen, R³ is halogen or preferably hydrogen, R⁴ is hydroxy, alkoxy, -OSO₂-Y (where Y is aryl which is either unsubstituted or substituted by alkyl or alkyl which is either unsubstituted or substituted by halogen), or preferably hydrogen, R⁵ is alkyl, R⁶ is hydrogen, alkyl, or preferably halogen, R⁴ and R⁶ may jointly represent =CH₂, R³ and R⁶ may jointly represent -S-, provided that where R⁴ and R⁶ are independent of each other, at least either one is hydrogen, exclusive of the case in which R¹ is amino, R² is hydrogen, R³ is hydrogen or halogen, R⁴ is hydrogen or hydroxy, R⁵ is alkyl, and R⁶ is hydrogen, and the pharmaceutically acceptable salt thereof.

Particularly preferred is the compound in which R¹ is amino, R² is hydrogen, R³ is hydrogen, R⁴ is hydrogen, R⁵ is methyl, and R⁶ is chlorine and the pharmaceutically acceptable salt thereof.

Among specific compounds of general formula [I], the compounds selected according to the following criteria selectively inhibit PDE isozymes I and IV. These compounds are hereinafter referred to collectively as Group C.

The compound in which R¹ is amino, R² is hydrogen, R³ is hydroxy, mercapto, or preferably -S-X (where X represents aryl which is either unsubstituted or substituted by halogen, or alkyl substituted by hydroxy or alkoxyalkoxy, preferably unsubstituted alkyl or cycloalkyl), R⁴ is hydrogen, alkoxy, or preferably hydroxy, R⁵ is alkyl, and R⁶ is hydrogen and the pharmaceutically acceptable salt thereof.

Particularly preferred is the compound in which R¹ is amino, R² is hydrogen, R³ is isopropylthio, R⁴ is hydroxy, R⁵ is ethyl, and R⁶ is hydrogen and its pharmaceutically acceptable salt.

The results of pharmacological tests for showing the usefulness of some representative species of the compound of the invention are set forth below.

The inhibitory activity of the compound against phosphodiesterase (PDE) isozymes I, II and IV was evaluated.

The PDE isozymes can be extracted and purified from various animal tissues but for expedient isolation of isozymes with high homogeneity in substantial amounts, the bovine urinary bladder or the bovine heart can be used as the source tissue. Thus, each isozyme can be isolated by the method which comprises homogenizing the source tissue in a suitable buffer, recovering a soluble fraction by a fractionation technique such as centrifugation, and purifying the soluble fraction by, for example, ion exchange chromatography or calmodulin-sepharose affinity chromatography.

The isozyme thus obtained is concentrated by ammonium sulfate precipitation or ultrafiltration and the concentrate is directly freezed and stored at -80°C, or lyophilized and then stored at a temperature not over -20°C, whereby the enzyme can be kept useful without deactivation for more than one year.

PDE isozymes I, II and IV were isolated from the bovine urinary bladder and PDE isozyme I from the bovine heart by the methods described in Reference Examples 34 and 35, and the effect of the compound of the present invention on the enzyme activity of each isozyme was investigated.

The assay of inhibitory activity was carried out in accordance with the method of Thompson and Appleman (W. J. Thompson, M. M. Appleman, Biochemistry, 10, p311 (1971)).

The following procedure was used for assaying the inhibitory activity against the bovine bladder-derived or heart-derived PDE isozyme I.

To 50 mM Tris-HCl buffer (pH 8.0) (buffer A) containing 10 mM magnesium chloride and 5 mM 2-mercaptoethanol were added 22,000 dpm [³H]-labeled cyclic AMP, 0.1-100 µM cyclic AMP, 66 ng/ml calmodulin (Boehringer-Mannheim-Yamanouchi), 3 mM calcium chloride, and 50 µg PDE isozyme I. All the concentrations indicated are final concentrations. To the mixture thus obtained was added a varying concentration of the test compound and the reaction mixture was adjusted to a final volume of 400 µl. The reaction was carried out at 30°C for 10 minutes, at the end of which time the reaction mixture was heated at 97°C for 5 minutes to deactivate the enzyme.

After the [³H]-5'-AMP produced by phosphodiesterase activity was decomposed to [³H]-adenosine by the procedure described in Reference Example 34, the radioactivity was measured and the enzyme activity was calculated.

The following procedure was used for assaying the inhibitory activity against the bovine bladder-derived PDE isozyme II.

To buffer A were added 22,000 dpm [³H]-labeled cyclic AMP, 0.1-100 µM cyclic AMP, 10 µM cyclic GMP, and 5 µg PDE isozyme II. To this mixture was added a varying concentration of the test compound and the final volume was adjusted to 400 µl. The reaction was carried out at 30°C for 10 minutes, at the end of which time the reaction mixture was heated at 97°C for 5 minutes to deactivate the enzyme.

The above reaction mixture was treated in the same manner as above and the enzyme activity was calculated.

The following procedure was used for assaying the inhibitory activity against the bovine bladder-derived PDE isozyme IV.

To buffer A were added 22,000 dpm [³H]-labeled cyclic AMP, 0.1-100 µM cyclic AMP, and 5 µg PDE isozyme IV. To this mixture was added a varying concentration of the test compound and the final volume was adjusted to 400 µl. The reaction was carried out at 30°C for 10 minutes, at the end of which time the reaction mixture was treated at 97°C for 5 minutes to deactivate the enzyme.

The above reaction mixture was treated in the same manner as above and the enzyme activity was calculated.

The inhibitory activity of the compounds of the present invention against the enzyme was shown in the following table as the 50% inhibitory concentration (IC₅₀, µM) with the enzyme activity in the absence of the test compound being taken as 100%, or the rate (%) of inhibition of enzyme activity in the presence of 100 µM or 1 mM of the test compound.

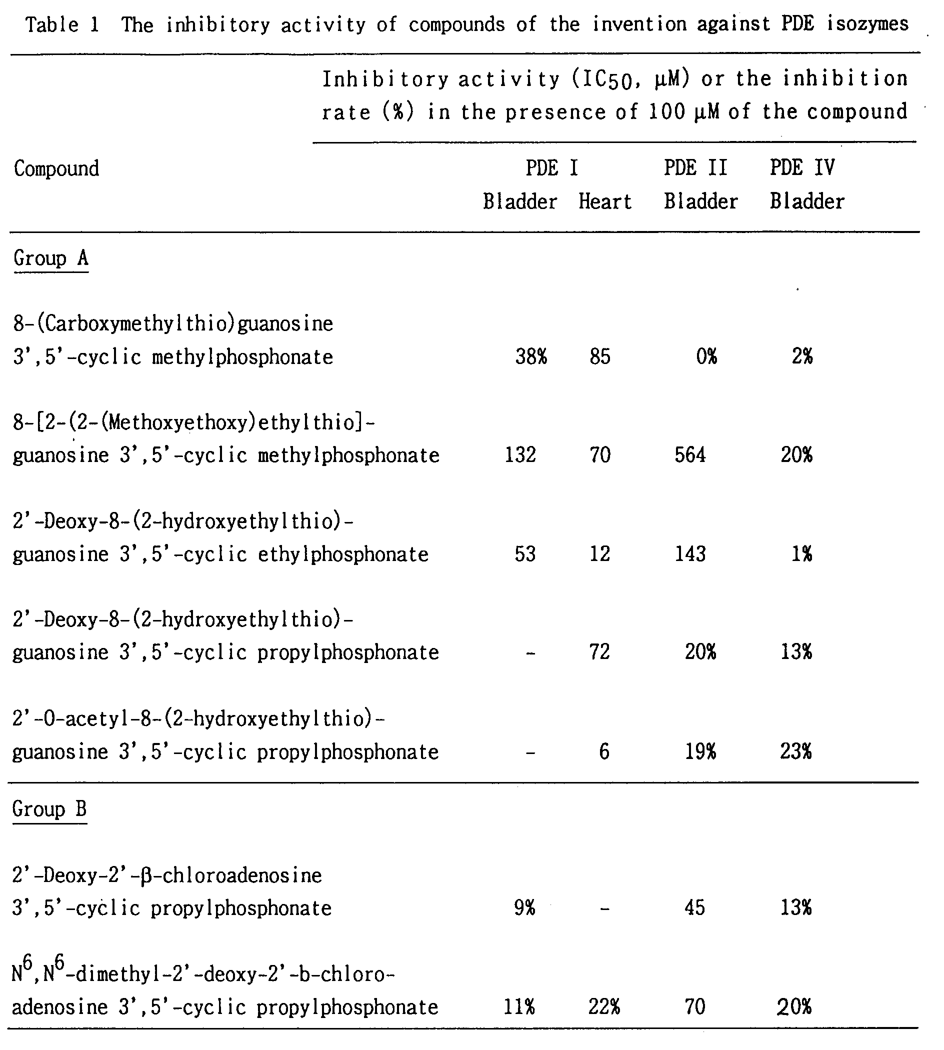

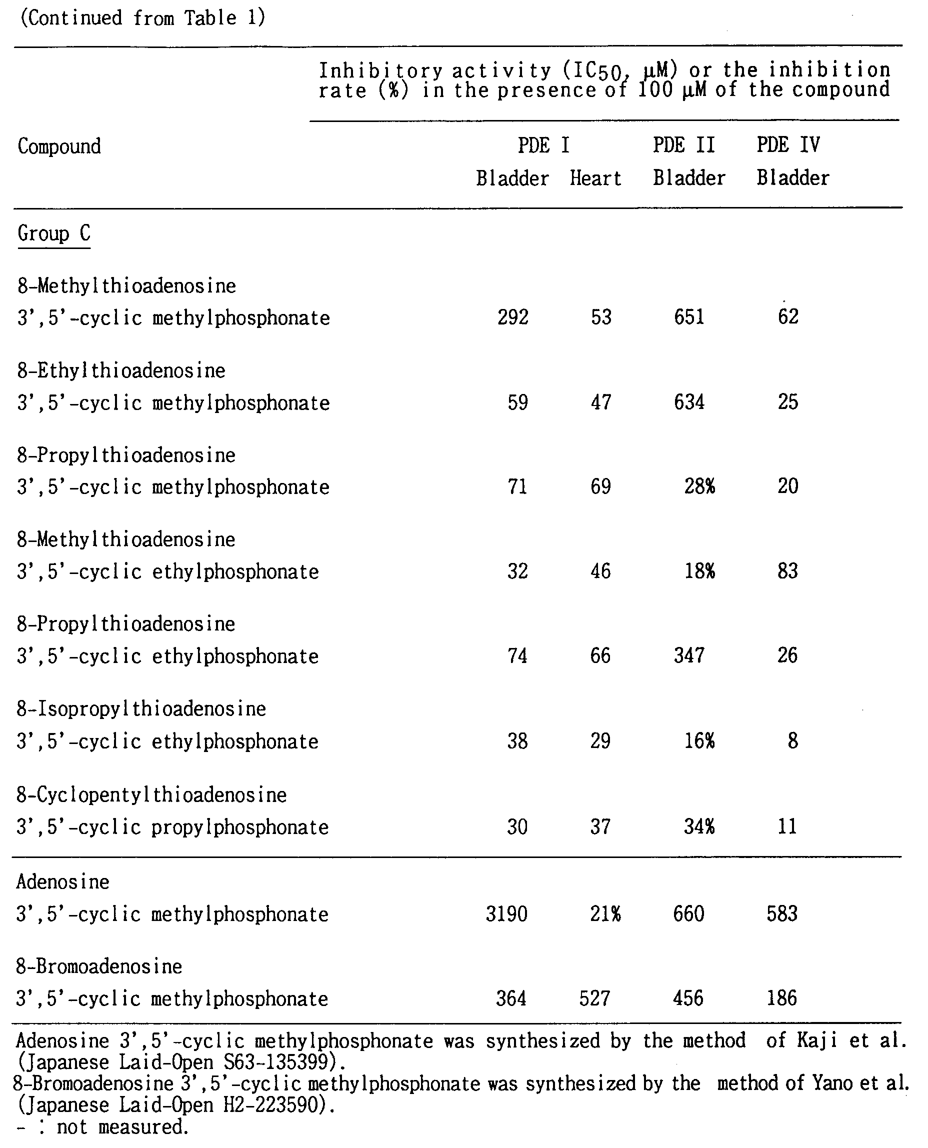

It is apparent from the above results that Group A compounds selectively inhibited PDE isozyme I, Group B compounds selectively inhibited PDE isozyme II, and Group C compounds selectively inhibited PDE isozymes I and IV. Moreover, the PDE isozyme selectivity and inhibitory activity of these compounds are higher than those of known cyclic nucleotide derivatives. In addition, the toxic potential of the compounds is low. Therefore, it is suggested that Group A compounds are useful typically as antidementia drugs, Group B compounds typically as antiarrhythmics, and Group C compounds typically as therapeutic drugs for bronchial asthma or antiinflammatory agents. Of course, uses for these groups of compounds are not limited to those mentioned but, by way of illustration, Group A compounds can be used as antiarrhythmics, therapeutic drugs for bronchial asthma, or antiinflammatory agents as well and Group B compounds are useful as antidementia drugs, therapeutic drugs for bronchial asthma, or antiinflammatory agents. Uses for Group C compounds are not limited to therapeutic drugs for bronchial asthma and antiinflammatory agents, either, but may be used as antidementia drugs or antiarrhythmics. In addition to the above uses, the compound of the present invention can be applicable to diverse medicinal uses, for example as a platelet aggregation inhibitor, an antihypertensive, a diuretic, or a muscle relaxant.

In using the compound of the present invention as a medicine, the compound can be administered to animals inclusive of human, either as it is or in the form of a pharmaceutical composition containing 0.1%-99.5%, preferably 0.5%-90%, of the compound and a medicinally acceptable nontoxic, inert carrier.

The carrier may be one or more kinds of solid, semisolid, or liquid diluents, fillers, and other formulation auxiliaries. The pharmaceutical composition is preferably administered in unit dosage forms. The pharmaceutical composition of the present invention can be administered orally, parenterally, locally (e.g. transdermally), or rectally. Of course, a dosage form suited for each route of administration should be used. A particularly preferred route is parenteral.

The dosage of a Group A compound for application as an antidementia drug is preferably selected with reference to patient conditions such as age and body weight, route of administration, nature and severity of illness, and other factors. Usually, for adults, the daily dose of 100 mg - 3 g/man, preferably 500 mg - 1 g/man, in terms of the active ingredient compound of the invention is general. A lower dosage may be sufficient in some cases, while a higher dosage may be necessary in other cases. It is preferable to give twice or thrice daily by dividing the daily dose in to two or three.

The dosage of a Group B compound for application as an antiarrhythmic drug is preferably selected with reference to patient conditions such as age and body weight, route of administration, nature and severity of illness, and other factors. Usually, for adults, the daily dose of 100 mg - 3 g/man, preferably 500 mg - 1 g/man, in terms of the active ingredient compound of the invention is general.

The dosage of a Group C compound for application as a therapeutic drug for bronchial asthma is preferably selected with reference to patient conditions such as age and body weight, route of administration, nature and severity of illness, and other factors. Usually, for adults, the daily dose of 100 mg - 3 g/man, preferably 500 mg - 1 g/man, in terms of the active ingredient compound of the invention is general.

The dosage of a Group C compound for application as an antiinflammatory drug is preferably selected with reference to patient conditions such as age and body weight, route of administration, nature and severity of illness, and other factors. Usually, for adults, the daily dose of 100 mg - 3 g/man, preferably 500 mg - 1 g/man, in terms of the active ingredient compound of the invention is general.

The dosage of a Group C compound for application as an antidementia drug is preferably selected with reference to patient conditions such as age and body weight, route of administration, nature and severity of illness, and other factors. Usually, for adults, the daily dose of 100 mg - 3 g/man, preferably 500 mg - 1 g/man, in terms of the active ingredient compound of the invention is general.

The compound [I] of the present invention can be produced typically by the following process. 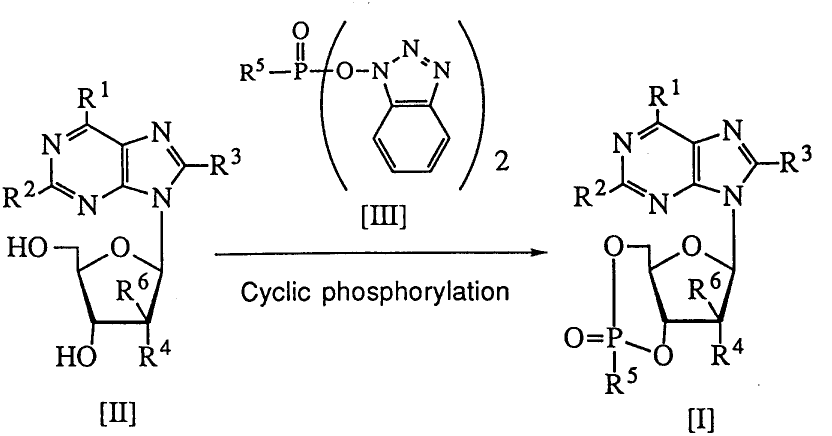 [Wherein R¹, R², R³, R⁴, R⁵, and R⁶ are the same as defined hereinbefore].

Compound [I] can be produced by subjecting compound [II] to cyclic phosphorylation reaction in accordance with the method described in the literature (Nucleic Acid Research, 14, p.2171, 1986).

Thus, compound [I] can be obtained by allowing compound [II] and compound [III] to stand together in a solvent inert to the reaction (e.g. dioxane, tetrahydrofuran (THF), N,N-dimethylformamide (DMF), methylene chloride, etc.) at 10-50°C for 30 minutes - 3 hours, then adding a base (e.g. an 1-alkylimidazole such as 1-methylimidazole, 1-ethylimidazole, etc., triethylamine, 4-dimethylaminopyridine, etc.), and allowing the mixture to stand further at 10-50°C for 5-24 hours.

The amount of compound [III] relative to compound [II] is preferably 1-2 molar equivalents. The amount of said base relative to compound [II] is preferably 2-10 molar equivalents.

However, in cases where R³ is hydroxy or mercapto, not only compound [I] but also certain byproduct compounds originating from side reactions are likely to be produced and, in such cases, the objective compound [I] will have to be isolated and purified by the general purification procedures described hereinafter.

The compound [Ia] that is the compound [I] of the present invention wherein R⁴ represents hydroxy and R⁶ represents hydrogen, can also be produced by the following process. 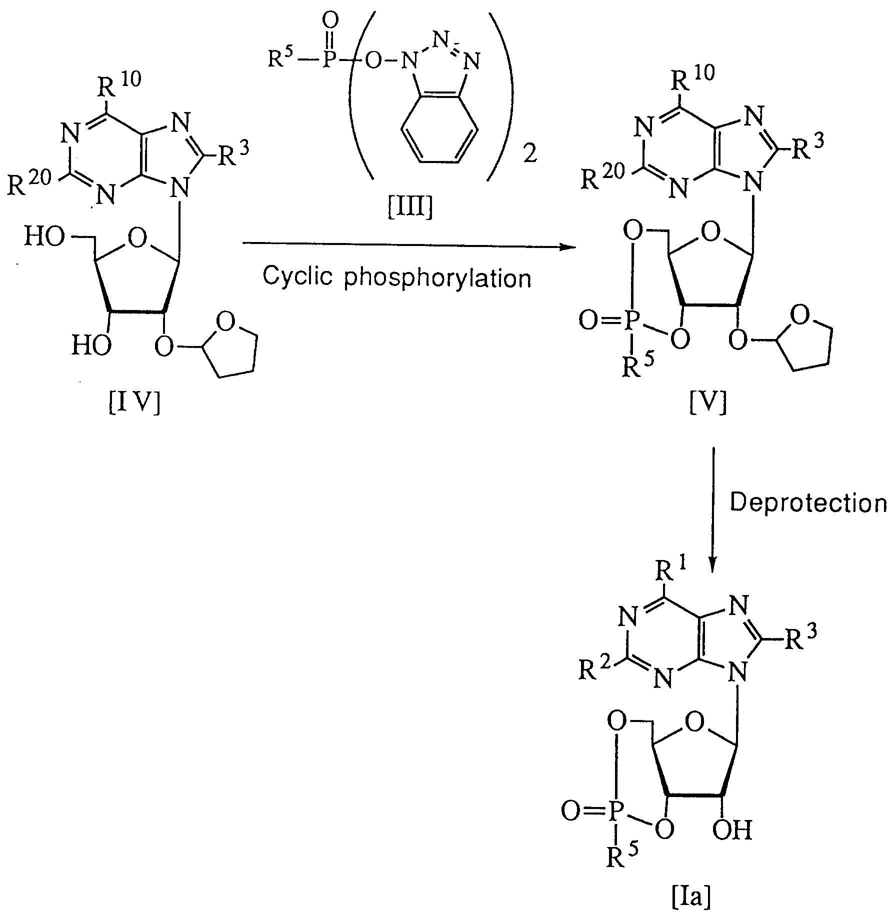 [Wherein R¹, R², R³, and R⁵ are the same as defined hereinbefore; R¹⁰ represents hydroxy, the same -NR¹¹R¹² as defined hereinbefore, or N-dimethoxytritylamino; R²⁰ represents hydrogen or N-dimethoxytrityl]

Compound [V] and compound [IV] can also be produced from compound [III] by a cyclic phosphorylation reaction similar to that described above for the production of compound [I].

Compound [Ia] can be produced by adding an acid of pH about 2-3 (e.g. trifluoroacetic acid, aqueous acetic acid, hydrochloric acid, etc.) to compound [V] at 10-50°C and allowing the mixture to stand for 5-24 hours to thereby remove the tetrahydrofuranyl group.

The compound [Ib] that is the compound [I] of the present invention wherein R⁴ represents acyloxy and R⁶ represents hydrogen, can also be produced by the following process. 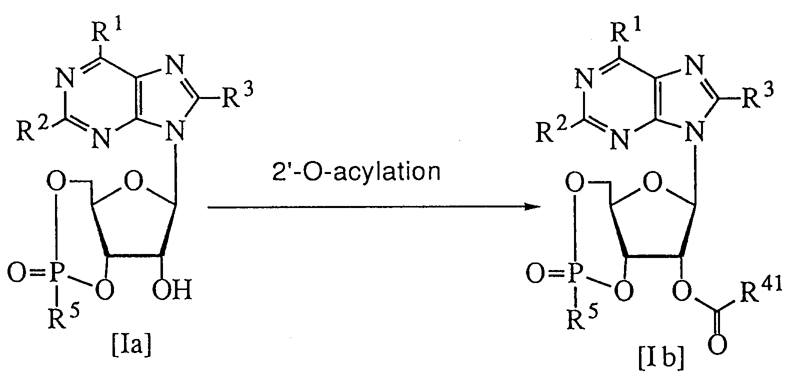 [Wherein R¹, R², R³, and R⁵ are the same as defined hereinbefore; R⁴¹ represents the alkyl moiety of the same acyloxy group as defined above for R⁴]

Compound [Ib] can be obtained by reacting compound [Ia] with an acid anhydride corresponding to R⁴¹ (e.g. acetic anhydride, propionic anhydride, valeric anhydride, etc.) in the presence of a catalyst amount of an organic base such as dimethylaminopyridine, triethylamine, in a solvent inert to the reaction (e.g. acetonitrile, dioxane, THF, DMF, methylene chloride, etc.) at 0°C - room temperature for 0.5-24 hours to thereby acylate only the 2'-position.

The amount of said organic base, e.g. triethylamine, relative to compound [Ia] is preferably 1-5 molar equivalents. The preferred amount of said acid anhydride relative to compound [Ia] is 1-2 molar equivalents.

The compound [Id] that is the compound [I] of the present invention wherein R³ represents halogen, can also be produced by the following process starting with compound [Ic] wherein R³ is hydrogen. 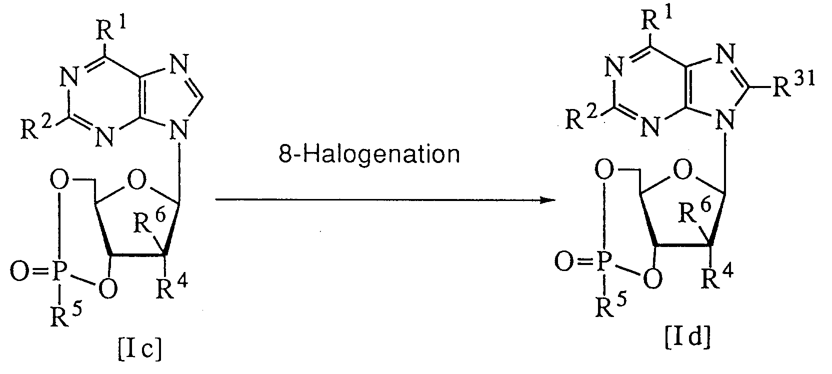 [Wherein R¹, R², R⁴, R⁵, and R⁶ are the same as defined hereinbefore; R³¹ represents the same halogen as R³ defined hereinbefore]

Compound [Id] can be produced by halogenating compound [Ic] in a suitable manner. For example, compound [Id] (R³¹=bromine) can be produced by reacting compound [Ic] with bromine in acetate buffer or water at 0°C - room temperature for 1-24 hours.

The amount of bromine relative to compound [Ic] is preferably 1-3 molar equivalents.

The compound [Ie] that is the compound [I] of the present invention wherein R³ represents hydroxy and R⁴ is tha same as defined hereinbefore but is not acyloxy or -OSO₂-Y, can also be produced by the following process starting from compound [Id'] wherein R³ is halogen. 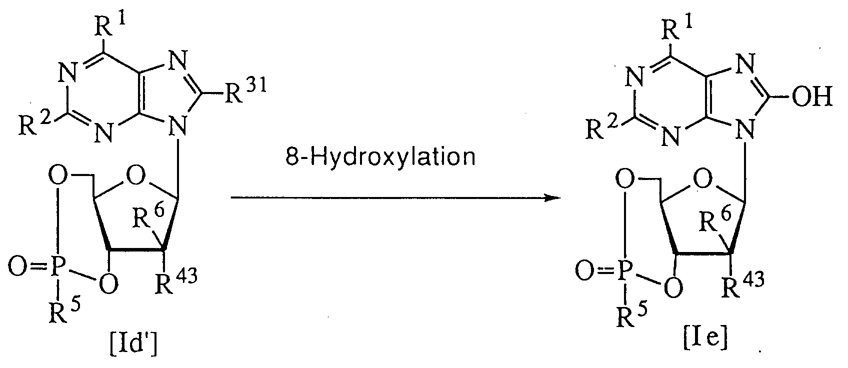 [Wherein R¹, R², R⁵, R⁶, and R³¹ are the same as defined hereinbefore; R⁴³ means the same as R⁴ defined hereinbefore but is not acyloxy or -OSO₂-Y]

Compound [Ie] can be produced by heating compound [Id'] in the presence of a large excess of a base (e.g. sodium acetate, potassium acetate, etc.) in acetic acid at 50°-120°C for 1-10 hours.

The amount of said base relative to compound [Id'] is preferably 10-20 molar equivalents.

The compound [If] that is the compound [I] of the present invention wherein R³ is mercapto, can also be produced from compound [Id] wherein R³ is halogen by the following process. 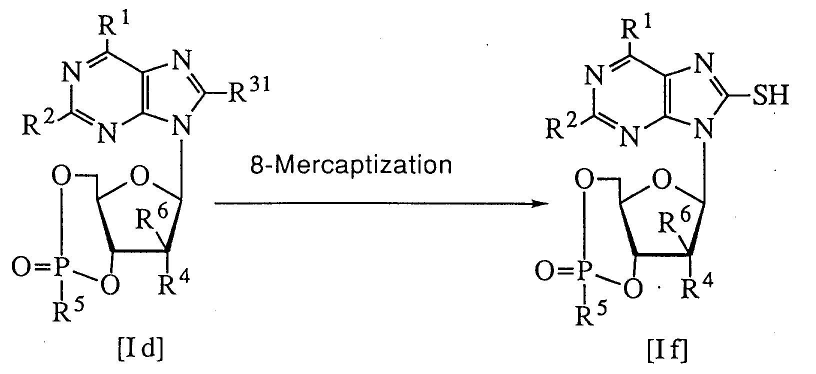 [Wherein R¹, R², R⁴, R⁵, R⁶, and R³¹ are the same as defined hereinbefore]

Compound [If] can be produced by refluxing compound [Id] and thiourea in an alcohol (e.g. ethanol, n-butanol, etc.) at 60-160°C for 0.5-24 hours.

The amount of thiourea relative to compound [Id] is preferably 2-20 molar equivalents.

The compound [Ig] that is the compound [I] of the present invention wherein R³ is -S-X where X represents an unsubstituted or substituted alkyl group (the substituent is selected from among hydroxy, carboxy, and alkoxyalkoxy) or a cycloalkyl group, can also be produced by the following process starting with compound [If] wherein R³ represents mercapto. 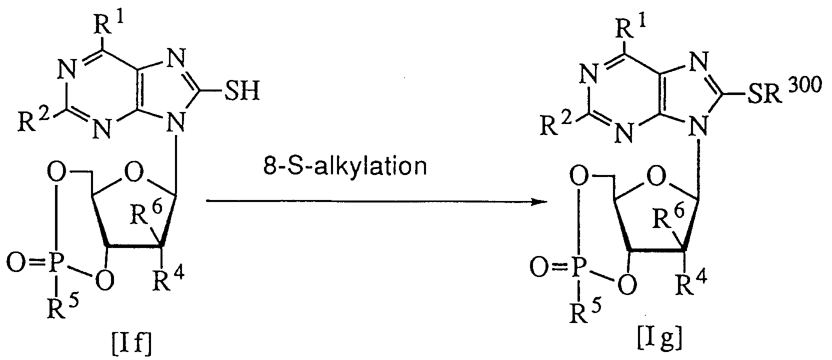 [Wherein R¹, R², R⁴, R⁵, and R⁶ are the same as defined hereinbefore; R³⁰⁰ represents the same unsubstituted or substituted alkyl (the substituent is selected from among hydroxy, carboxy, and alkoxyalkoxy) or cycloalkyl as defined for X].

Compound [Ig] can be produced by reacting compound [If] with an alkyl halide (e.g. methyl iodide, ethyl iodide, propyl iodide, isopropyl iodide, cyclopentyl bromide, 2-methoxyethoxy bromide, hexyl bromide, carboxymethyl bromide, 2-hydroxyethyl bromide, 3-hydroxypropyl bromide, etc.) in the presence of a base (an inorganic base such as potassium carbonate or an organic base such as triethylamine) in a polar solvent (e.g. DMF, methanol, ethanol, etc.) at 0°-100°C for 1-24 hours.

The amounts of said base and alkyl halide relative to compound [If] are preferably 1-5 molar equivalents.

The compound [Ih] that is the compound [I] of the present invention wherein R³ represents -S-X where X is an unsubstituted or halogen-substituted aryl group, can also be produced by the following process starting with compound [Id] wherein R³ represents halogen. 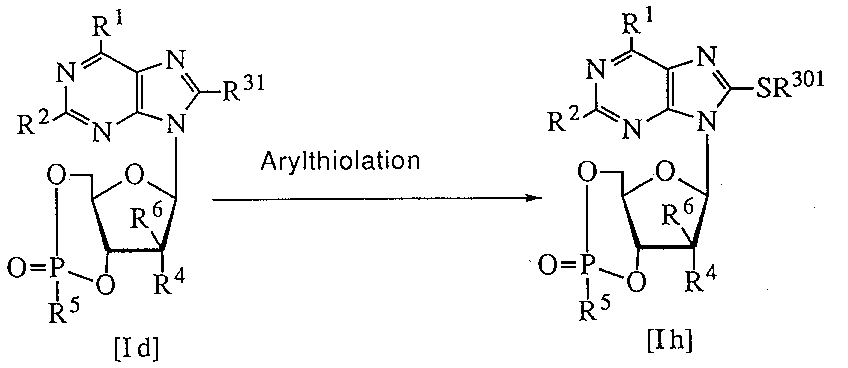 [Wherein R¹, R², R⁴, R⁵, R⁶, and R³¹ are the same as defined hereinbefore; R³⁰¹ represents the same unsubstituted or halogen-substituted aryl as mentioned for X hereinbefore]

Compound [Ih] can be produced by reacting compound [Id] with an arylmercaptan (e.g. thiophenol, 4-fluorothiophenol, 4-bromothiophenol, 4-chlorothiophenol, 4-iodothiophenol, 2-fluorothiophenol, 2-bromothiophenol, 3-chlorothiophenol, 3-iodothiophenol, etc.) in the presence of a base (an inorganic base such as potassium carbonate or an organic base such as triethylamine) in a polar solvent (e.g. DMF, methanol, ethanol, etc.) at 0°-100°C for 1-24 hours.

The amounts of said base and arylmercaptan relative to compound [Id] are preferably 1-5 molar equivalents.

The compound [Ij] that is the compound [I] of the present invention wherein R³ represents hydrogen or halogen, R⁴ represents -OSO₂-Y where Y represents an unsubstituted or alkyl-substituted aryl group or an unsubstituted or halogen-substituted alkyl group, and R⁶ is hydrogen, can also be produced by the following process starting with compound [Ii] wherein R⁴ is hydroxy. 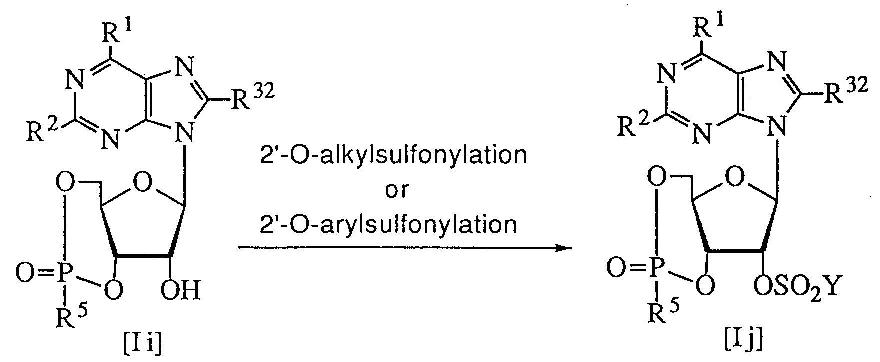 [Wherein R¹, R², R⁵, and Y are the same as defined hereinbefore; R³² represents hydrogen or halogen]

Compound [Ij] can be produced by reacting compound [Ii] with an alkylsulfonyl chloride (e.g. methanesulfonyl chloride, trifluoromethanesulfonyl chloride, etc.), an arylsulfonyl chloride (e.g. p-toluenesulfonyl chloride etc.), or an alkylsulfonic anhydride (e.g. methanesulfonic anhydride, trifluoromethanesulfonic anhydride, etc.) in the presence of a large excess of an organic base (e.g. pyridine, triethylamine, 4-dimethylaminopyridine, said 1-alkylimidazole, etc.) either in an aprotic solvent (e.g. methylene chloride, toluene, etc.) or in the absence of a solvent at 0°-50°C for 10 minutes ∼ 24 hours.

The amount of said alkylsulfonyl chloride, arylsulfonyl chloride or alkylsulfonic anhydride relative to compound [Ii] is preferably 1-5 molar equivalents.

The compound [Ik] that is the compound [I] of the present invention wherein R⁶ represents halogen, R⁴ represents hydrogen and R³ represents hydrogen or halogen, can also be produced from compound [Ij] by the following process. 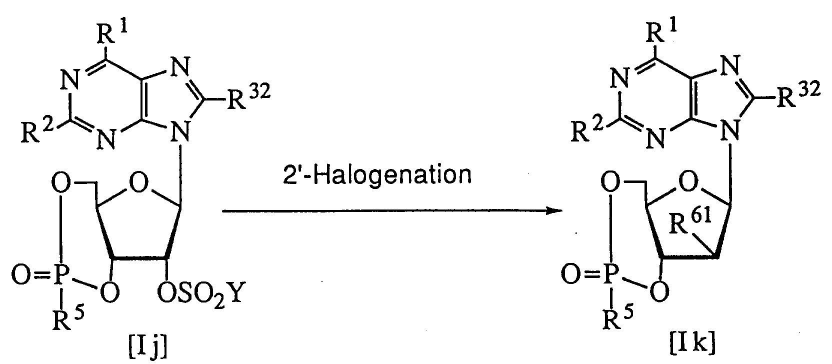 [Wherein R¹, R², R⁵, Y, and R³² are the same as defined hereinbefore; R⁶¹ represents hydrogen or halogen]

Compound [Ik] can be produced by reacting compound [Ij] with a large excess of an alkali metal halide (e.g. lithium chloride, lithium bromide, cesium fluoride, potassium iodide, etc.) in a polar solvent (e.g. DMF, hexamethylphosphoric triamide, etc.) at 10°-50°C for 5 hours - 3 days.

The compound [Ik'] that is the compound [Ik] wherein R³² represents hydrogen, can also be produced by the following process starting with compound [Ik''] (compound [Ik] wherein R³² represents chlorine, bromine or iodine). 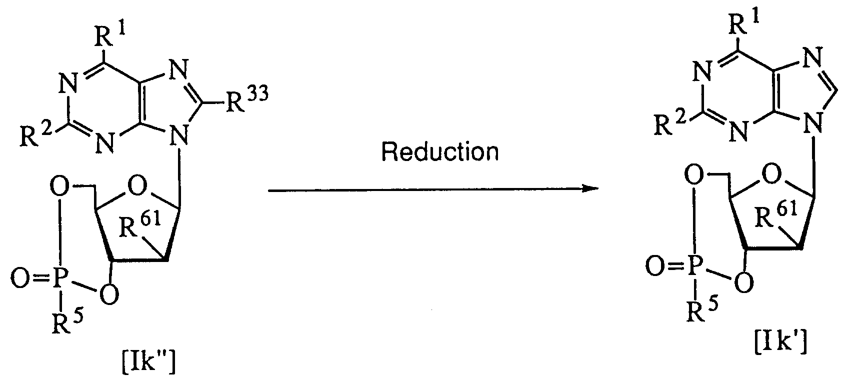 [Wherein R¹, R², R⁵, and R⁶¹ are the same as defined hereinbefore; R³³ represents chlorine, bromine or iodine]

Compound [Ik'] can be produced by reducing compound [Ik''] with hydrogen gas at 1-5 atmospheres in the presence of 1-20 weight % palladium-on-carbon in an alcohol (e.g. methanol, ethanol, etc.) at 10°-50°C for 1-24 hours to thereby selectively reduce the halogen atom on the nucleotide base.

The compound [Ij'] that is the compound of formula [Ij] wherein R³² represents hydrogen, can also be produced by the following process starting with compound [Ij''] (compound [Ij] wherein R³² represents chlorine, bromine or iodine). 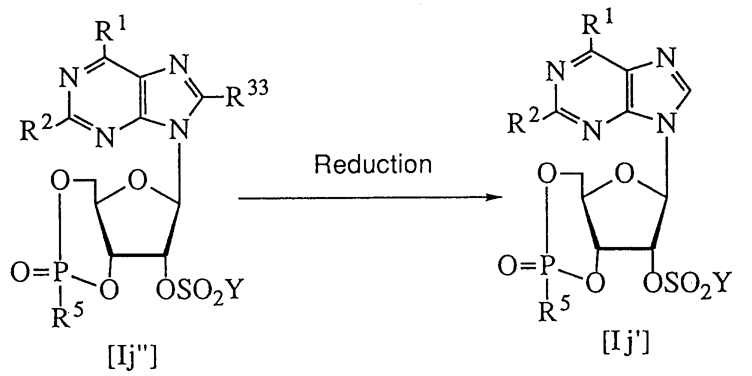 [Wherein R¹, R², R⁵, R³³, and Y are the same as defined hereinbefore]

Compound [Ij'] can be produced from compound [Ij''] in the same manner as described above for the production of compound [Ik'].

The compound [In] that is the compound [I] wherein R³ and R⁴ each represents hydrogen and R⁶ represents methyl, can be produced by the following process starting with compound [Im]. 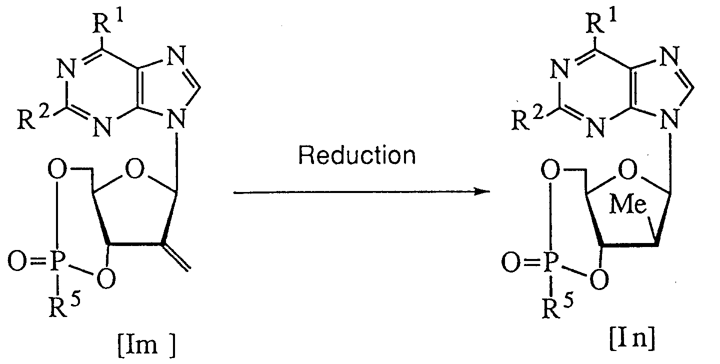 [Wherein R¹, R², and R⁵ are the same as defined hereinbefore]

Compound [In] can be produced from compound [Im] in the same manner as the above-mentioned production of compound [Ik']. The catalyst that can be used includes various acids such as acetic acid, hydrochloric acid and sulfuric acid.

The compound [Io] that is the compound [I] of the present invention wherein R³ represents methyl, can also be produced by the following process starting with compound [Ic]. 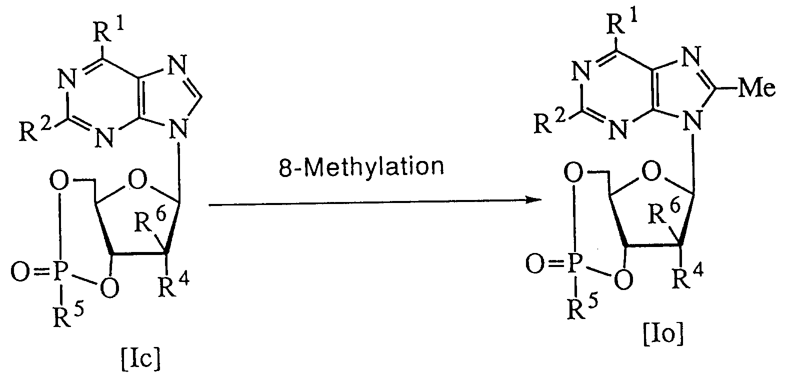 [Wherein R¹, R², R⁴, R⁵, and R⁶ are the same as defined hereinbefore]

Compound [Io] can be produced by reacting compound [Ic] with 3-10 molar equivalents of ferrous sulfate and 3-10 molar equivalents of tert-butyl hydroperoxide in an aqueous solution of sulfuric acid at 0°C - room temperature for 0.5-10 hours.

The amounts of ferrous sulfate and tert-butyl hydroperoxide relative to compound [Ic] are preferably 3-10 molar equivalents.

The compound [Iq] that is the compound [I] of the invention wherein both R¹ and R² represent hydroxy, can also be produced by the following process starting with compound [Ip]. 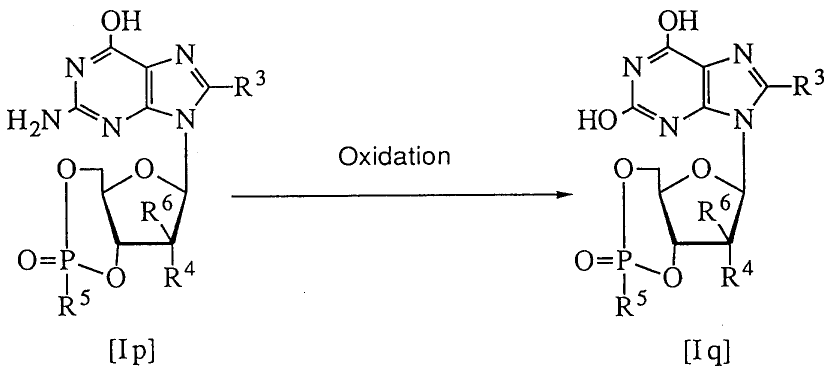 [Wherein R³, R⁴, R⁵, and R⁶ are the same as defined hereinbefore]

Compound [Iq] can be produced by oxidizing compound [Ip] with a large excess of sodium nitrite in an acidic aqueous solution (e.g. aqueous acetic acid solution, diluted hydrochloric acid, etc.) at 0°C - room temperature for 1-24 hours.

The compound [I] or salt thus produced can be isolated and purified by known procedures such as solvent extraction, pH adjustment, redistribution, concentration, crystallization, recrystallization, and chromatography.

The starting compounds [II], [III] and [IV] used in the production of the compound of the present invention are either known compounds or can be produced by processes analogous with known processes (e.g. Nucleic Acid Res., 14, p. 2171, 1986; Tetrahedron, 40, p. 153, 1984), for example as shown in the following reference examples.

### BEST MODE OF PRACTICING THE INVENTION

The following reference and working examples relating to the production of compounds are intended to describe the present invention in further detail.

### Reference Example 1

### Adenosine 3',5'-cyclic methylphosphonate

To 5 g of N-dimethoxytrityl-2'-O-tetrahydrofuranyladenosine was added 100 ml of 0.11 M methyl-O,O-bis(1-benzotriazolyl)phosphonate/dioxane solution at room temperature. After 20 minutes, 3 g of 1-methylimidazole was added and the mixture was stirred at room temperature overnight. This reaction mixture was concentrated and the residue was dissolved in ethyl acetate, washed with 0.2N aqueous monopotassium phosphate solution, and concentrated to dryness. The residue was dissolved in 200 ml of dioxane and the solution was acidified to pH 2 with 0.1N hydrochloric acid and stirred overnight. This reaction mixture was diluted with water and washed with ethyl ether and the aqueous layer was concentrated. The residue was subjected to reversed phase column chromatography (CAPCELL C₁₈ SG120, 30 µm, manufactured by Shiseido) and elution was carried out with methanol-water (8%-15% gradient) to provide 1.24 g of the title compound as white powders.
m.p. 220-225°C (decomp.)

### Reference Example 2

### Adenosine 3',5'-cyclic ethylphosphonate

Using N-dimethoxytrityl-2'-O-tetrahydrofuranyladenosine and ethyl O,O-bis(1-benzotriazolyl)phosphonate, the procedure of Reference Example 1 was otherwise repeated to provide the title compound.
m.p. 160-165°C

### Reference Example 3

### Adenosine 3',5'-cyclic propylphosphonate

Using N-dimethoxytrityl-2'-O-tetrahydrofuranyladenosine and propyl O,O-bis(1-benzotriazolyl)phosphonate, the procedure of Reference Example 1 was otherwise repeated to provide the title compound.
m.p. 115-200°C

### Reference Example 4

### 2'-Deoxyadenosine 3',5'-cyclic methylphosphonate

Using N-dimethoxytrityl-2'-deoxyadenosine and methyl O,O-bis(1-benzotriazolyl)phosphonate, the procedure of Reference Example 1 was otherwise repeated to provide the title compound. m.p. 222-225°C (decomp.)

### Reference Example 5

### 2'-Deoxyadenosine 3',5'-cyclic ethylphosphonate

Using N-dimethoxytrityl-2'-deoxyadenosine and ethyl O,O-bis(1-benzotriazolyl)phosphonate, the procedure of Reference Example 1 was otherwise repeated to provide the title compound.
m.p. 227-234°C (decomp.)

### Reference Example 6

### 2'-Deoxyadenosine 3',5'-cyclic propylphosphonate

Using N-dimethoxytrityl-2'-deoxyadenosine and propyl O,O-bis(1-benzotriazolyl)phosphonate, the procedure of Reference Example 1 was otherwise repeated to provide the title compound.
m.p. 220-223°C

### Reference Example 7

### Guanosine 3',5'-cyclic methylphosphonate

Using N-dimethoxytrityl-2'-O-tetrahydrofuranylguanosine and methyl O,O-bis(1-benzotriazolyl)phosphonate, the procedure of Reference Example 1 was otherwise repeated to provide the title compound.
m.p. 223-225°C (decomp.)

### Reference Example 8

### Guanosine 3',5'-cyclic ethylphosphonate

Using N-dimethoxytrityl-2'-O-tetrahydrofuranylguanosine and ethyl O,O-bis(1-benzotriazolyl)phosphonate, the procedure of Reference Example 1 was otherwise repeated to provide the title compound.
m.p. 225-231°C (decomp.)

### Reference Example 9

### Guanosine 3',5'-cyclic propylphosphonate

Using N-dimethoxytrityl-2'-O-tetrahydrofuranylguanosine and propyl O,O-bis(1-benzotriazolyl)phosphonate, the procedure of Reference Example 1 was otherwise repeated to provide the title compound.
m.p. 237-243°C (decomp.)

### Reference Example 10

### 2'-Deoxyguanosine 3',5'-cyclic methylphosphonate

Using N-dimethoxytrityl-2'-deoxyguanosine and methyl O,O-bis(1-benzotriazolyl)phosphonate, the procedure of Reference Example 1 was otherwise repeated to provide the title compound.
m.p. 203-218°C (decomp.)

### Reference Example 11

### 2'-Deoxyguanosine 3',5'-cyclic ethylphosphonate

Using N-dimethoxytrityl-2'-deoxyguanosine and ethyl O,O-bis(1-benzotriazolyl)phosphonate, the procedure of Reference Example 1 was otherwise repeated to provide the title compound. m.p. 215-221°C (decomp.)
Reference Example 12

### 2'-Deoxyguanosine 3',5'-cyclic propylphosphonate

Using N-dimethoxytrltyl-2'-deoxyguanosine and propyl O,O-bis(1-benzotriazolyl)phosphonate, the procedure of Reference Example 1 was otherwise repeated to provide the title compound.
m.p. 215-224°C (decomp.) Reference Example 13

### 8-Bromoadenosine 3',5'-cyclic methylphosphonate

In 20 ml of 0.5N sodium acetate buffer (pH 4) was dissolved 0.93 g of the adenosine 3',5'-cyclic methylphosphonate obtained in Reference Example 1 followed by addition of 30 ml of aqueous bromine solution and the mixture was stirred for 1 hour. This reaction mixture was concentrated to dryness and the residue was subjected to reversed phase column chromatography, elution being carried out with 30% methanol-water to provide 1.1 g of the title compound as white powders.
m.p. 215-220°C (decomp.)

### Reference Example 14

### 8-Bromoadenosine 3',5'-cyclic ethylphosphonate

Using the adenosine 3',5'-cyclic ethylphosphonate obtained in Reference Example 2, the procedure of Reference Example 13 was otherwise repeated to provide the title compound. m.p. 200-210°C (decomp.)

### Reference Example 15

### 8-Bromoadenosine 3',5'-cyclic propylphosphonate

Using the adenosine 3',5'-cyclic propylphosphonate obtained in Reference Example 3, the procedure of Reference Example 13 was otherwise repeated to provide the title compound. m.p. 160-163°C, 205°C (decomp.)

### Reference Example 16

### 8-Bromo-2'-deoxyadenosine 3',5'-cyclic methylphosphonate

Using the 2'-deoxyadenosine 3',5'-cyclic methylphosphonate obtained in Reference Example 4, the procedure of Reference Example 13 was otherwise repeated to provide the title compound.
m.p. 195-200°C (decomp.)

### Reference Example 17

### 8-Bromo-2'-deoxyadenosine 3',5'-cyclic ethylphosphonate

Using the 2'-deoxyadenosine 3',5'-cyclic ethylphosphonate obtained in Reference Example 5, the procedure of Reference Example 13 was otherwise repeated to provide the title compound.
m.p. 198-203°C (decomp.)

### Reference Example 18

### 8-Bromo-2'-deoxyadenosine 3',5'-cyclic propylphosphonate

Using the 2'-deoxyadenosine 3',5'-cyclic propylphosphonate obtained in Reference Example 6, the procedure of Reference Example 13 was otherwise repeated to provide the title compound.
m.p. 191-193°C Reference Example 19

### 8-Bromoguanosine 3',5'-cyclic methylphosphonate

Using the guanosine 3',5'-cyclic methylphosphonate obtained in Reference Example 7, the procedure of Reference Example 13 was otherwise repeated to provide the title compound. m.p. 215-220°C (decomp.)

### Reference Example 20

### 8-Bromoguanosine 3',5'-cyclic ethylphosphonate

Using the guanosine 3',5'-cyclic ethylphosphonate obtained in Reference Example 8, the procedure of Reference Example 13 was otherwise repeated to provide the title compound.

### Reference Example 21

### 8-Bromoguanosine 3',5'-cyclic propylphosphonate

Using the guanosine 3',5'-cyclic propylphosphonate obtained in Reference Example 9, the procedure of Reference Example 13 was otherwise repeated to provide the title compound.

### Reference Example 22

### 8-Bromo-2'-deoxyguanosine 3',5'-cyclic methylphosphonate

Using the 2'-deoxyguanosine 3',5'-cyclic methylphosphonate obtained in Reference Example 10, the procedure of Reference Example 13 was otherwise repeated to provide the title compound.
m.p. 216-221°C (decomp.)

### Reference Example 23

### 8-Bromo-2'-deoxyguanosine 3',5'-cyclic ethylphosphonate

Using the 2'-deoxyguanosine 3',5'-cyclic ethylphosphonate obtained in Reference Example 11, the procedure of Reference Example 13 was otherwise repeated to provide the title compound.

### Reference Example 24

### 8-Bromo-2'-deoxyguanosine 3',5'-cyclic propylphosphonate

Using the 2'-deoxyguanosine 3',5'-cyclic propylphosphonate obtained in Reference Example 12, the procedure of Reference Example 13 was otherwise repeated to provide the title compound.

### Reference Example 25

### 2'-O-acetylguanosine 3',5'-cyclic methylphosphonate

To a suspension of 440.5 mg of the guanosine 3',5'-cyclic methylphosphonate obtained in Reference Example 7 and 12.2 mg of 4-dimethylaminopyridine in 20 ml of acetonitrile, were added 0.16 ml of acetic anhydride and 0.27 ml of triethylamine and the mixture was stirred for 1 hour. The precipitate that formed was collected by filtration to provide 350 mg of the title compound.
m.p. 245-250°C (decomp.)

### Reference Example 26

### 2'-O-acetylguanosine 3',5'-cyclic propylphosphonate

Using the guanosine 3',5'-cyclic propylphosphonate obtained in Reference Example 9, the procedure of Reference Example 25 was otherwise repeated to provide the title comopund. m.p. 245-256°C (decomp.)

### Reference Example 27

### 2'-O-propionylguanosine 3',5'-cyclic propylphosphonate

Using the guanosine 3',5'-cyclic propylphosphonate obtained in Reference Example 9 and propionylic anhydride, the procedure of Reference Example 25 was otherwise repeated to provide the title compound.
m.p. 230-232°C (decomp.)

### Reference Example 28

### 2'-O-acetyl-8-bromoguanosine 3',5'-cyclic methylphosphonate

In 10 ml of water was suspended 350 mg of the 2'-O-acetylguanosine 3',5'-cyclic methylphosphonate obtained in Reference Example 25, followed by addition of 10 ml of aqueous solution of bromine, and the mixture was stirred for 30 minutes. This reaction mixture was concentrated to dryness and the water was removed azeotropically with toluene to provide 502.7 mg of the title compound. m.p. 235-240°C (decomp.)

### Reference Example 29

### 2'-O-acetyl-8-bromoguanosine 3',5'-cyclic propylphosphonate

Using the 2'-O-acetylguanosine 3',5'-cyclic propylphosphonate obtained in Reference Example 26, the procedure of Reference Example 28 was otherwise repeated to provide the title compound.
m.p. 220-230°C (decomp.)

### Reference Example 30

### 2'-O-propionyl-8-bromoguanosine 3',5'-cyclic propylphosphonate

Using the 2'-O-propionylguanosine 3',5'-cyclic propylphosphonate obtained in Reference Example 27, the procedure of Reference Example 28 was otherwise repeated to provide the title compound.

### Reference Example 31

### N⁶,N⁶-dimethyl-2'-O-tetrahydrofuranyladenosine

In 20 ml of DMF were dissolved 4.31 g of N⁶,N⁶-dimethyladenosine and 3 g of imidazole followed by dropwise addition of 4.73 g of 1,3-dichlorotetraisopropyldisiloxane and the mixture was stirred for 3 hours. This reaction mixture was poured in iced water and extracted with ethyl acetate. The extract was washed with water and the solvent was distilled off to get 7.97 g of solid matter. This solid was dissolved in 80 ml of dioxane. Then, 20 g of 1,2-dihydrofuran and 4 g of pyridinium p-toluenesulfonate were added to the above solution and the mixture was stirred for 5 days. To this reaction mixture was added a mixture (4:1) of methylene chloride and pyridine (200 ml), followed washing with saturated sodium hydrogen carbonate solution, the solvent was distilled off. The residue was subjected to silica gel column chromatography and elution was carried out with 0.5% pyridine-25% n-hexane-ethyl acetate to get 5.67 g of colorless oily substance. This substance was dissolved in 20 ml of tetrahydrofuran followed by addition of 1 M tetrabutylammonium fluoride/THF and the mixture was stirred for 30 minutes. This reaction mixture was diluted with methylene chloride and washed with water and the solvent was distilled off. The residue was subjected to silica gel column chromatography and elution was carried out with 5% methanol-methylene chloride to provide 3.02 g of colorless foamy solid.
m.p. 230-235°C (decomp.)

### Reference Example 32

### 8-(tert-Butylthio)-2'-deoxyadenosine

In 30 ml of DMF was dissolved 3 g of 8-bromo-2'-deoxyadenosine followed by addition of 2.52 g of potassium carbonate and 12 ml of a solution of 0.89 M tert-butylmercaptan/DMF and the mixture was stirred at 100°C for 2 hours. This reaction mixture was concentrated and the residue was subjected to silica gel column chromatography using 5% methanol-methylene chloride as eluent to provide 2.05 g of the title compound.
m.p. 170-172°C

### Reference Example 33

### Phenyl-O,O-bis(1-benzotriazolyl)phosphonate

In 50 ml of dioxane were dissolved 2.05 g of 1-hydroxybenzotriazole and 1.5 ml of pyridine followed by dropwise addition of 15 ml of a solution of 1.46 g phenylphosphonic dichloride in dioxane and the mixture was stirred at room temperature for 2 hours. The precipitate was then filtered off to provide a solution of the title compound (0.11 M) in dioxane as a filtrate.

### Reference Example 34

### Preparation of PDE isozymes I, II, and IV from the bovine urinary bladder

Starting with bovine urinary bladders, PDE isozymes I, II, and IV were prepared in accordance with the method of R. E. Weishaar et al. (Biochemical Pharmacology, 35, p. 787 (1986)) and the method of B. E. Lavan et al. (Biochemical Pharmacology, 38, p. 4123 (1989)).

Using a homogenizer (Nissei Homogenizer AM-12), 30 g of bovine urinary bladder smooth muscle was evenly disrupted in 10 volumes (v/v) of buffer B (10 mM Tris-HCl buffer (pH 7.5) containing 2 mM MgCl₂, 1 mM dithiothreitol (DTT), 50 µM phenylmethanesulfonyl fluoride (PMSF), and 2 mM benzamidine) in 5 disruption cycles of 10,000 rpm x 30 seconds at 1-minute intervals and, then, centrifuged at 1,000 x g for 10 minutes. The resulting supernatant was filtered through a double wad of gauze, centrifuged at 100,000 x g for 60 minutes, and the precipitate was removed to get a soluble fraction.

To this soluble fraction was added solid ammonium sulfate to provide 25% saturation and the mixture was stirred for 30 minutes and then centrifuged at 10,000 x g for 20 minutes. To the resultant supernatant was added a further amount of solid ammonium sulfate to 65% saturation, which was stirred for 30 minutes and then centrifuged at 10,000 x g for 20 minutes. The resulting precipitate was dissolved in 5 ml of buffer C (20 mM Bis-tris-acetate buffer (pH 6.5) containing 5 mM DTT, 2 mM benzamidine, 2 mM EDTA, 50 µM PMSF, and 50 mM sodium acetate) and this fraction (containing 100 mg of protein) was dialyzed against 200 volumes of buffer C twice one hour apart and, then, dialyzed again overnight. After the insoluble fraction was removed by centrifugation (10,000 x g for 20 minutes), the soluble fraction was applied to a Mono-Q ion exchange column (Pharmacia, 10 mm x 100 mm) equilibrated with buffer C beforehand. After the column was washed with 60 ml of buffer C, PDE isozymes were eluted on a linear sodium acetate gradient from 0.05 M to 1 M in 240 ml of buffer C. The eluate was collected in 4 ml fractions and PDE activity in each fraction was measured. The above procedure was consistently carried out at 4°C.

Enzyme activity in each resultant fraction was measured in accordance with the method of Thompson and Appleman (W. J. Thompson, M. M. Appleman, Biochemistry, 10, p.311, 1971). Activity assays were carried by 4 different procedures.

Cyclic AMP-phosphodiesterase activity was assayed by the following procedure.

To 50 mM Tris-HCl buffer (pH 8.0) (buffer A) containing magnesium chloride and 2-mercaptoethanol at the final concentrations of 10 mM and 5 mM, respectively, were added 22,000 dpm [³H]-labeled cyclic AMP, 1 µM cyclic AMP, and 50 µl of the column fraction to make final volume to 400 µl. The reaction was conducted at 30°C for 10 minutes and the reaction mixture was then treated at 97°C for 5 minutes to deactivate the enzyme.

After the reaction mixture was cooled in iced water, 50 µg of snake venom (Sigma) was added and the reaction was carried out at 30°C for 10 minutes so that the [³H]5'-AMP produced by phosphodiesterase activity was decomposed to [³H]adenosine. The reaction mixture was then treated at 97°C for 5 minutes to deactivate the enzyme. To this reaction mixture was added 1 ml of a suspension of AG1-X2 resin (Bio-Rad) in 3 volumes of buffer A and the mixture was centrifuged at 1,000 x g for 10 minutes. A 400 µl portion of the supernatant was taken and mixed with 10 ml of a scintillator (Packard, Emulsifier Scintillator 299) and the radioactivity of [³H]adenosine was measured with an Aloka liquid scintillation counter and the enzyme activity was calculated.

Cyclic GMP-stimulating cyclic AMP-phosphodiesterase was assayed by the following procedure.

To buffer A were added 22,000 dpm [³H]-labeled cyclic AMP, 1 µM cyclic AMP, 10 µM cyclic GMP, and 50 µl of the column fraction to make final volume to 400 µl. The reaction was carried out at 30°C for 10 minutes and the reaction mixture was then treated at 97°C for 5 minutes to deactivate the enzyme.

This reaction mixture was treated in the same manner as described previously and the radioactivity of [³H]adenosine was measured to calculate the enzyme activity.

Cyclic GMP-phosphodiesterase activity was assayed by the following procedure.

To buffer A were added 22,000 dpm [³H]-labeled cyclic GMP, 1 µM cyclic GMP, and 10 µl of the column fraction to make final volume to final volume to 400 µl. The reaction was carried out at 30°C for 10 minutes and the reaction mixture was then treated at 97°C for 5 minutes to deactivate the enzyme.

After the reaction mixture was cooled in iced water, 50 µg of snake venom (Sigma) was added and the reaction was carried out at 30°C for 10 minutes, whereby the [³H]5'-GMP produced by phosphodiesterase activity was decomposed to [³H]guanosine. The reaction mixture was then treated at 97°C for 5 minutes to deactivate the enzyme. This reaction mixture was treated in the same manner as above and the radioactivity of [³H]guanosine was measured to calculate the enzyme activity.

Calcium/calmodulin-dependent cyclic GMP-phosphodiesterase activity was assayed by the following procedure.

To a mixture of buffer A, 22,000 dpm [³H]-labeled cyclic GMP, 1 µM cyclic GMP, 66 ng/ml calmodulin (Boehringer Mannheim-Yamanouchi), and 3 mM calcium chloride was added 10 µl of the column fraction to make 400 µl. The reaction was carried out at 30°C for 10 minutes and then treated at 97°C for 5 minutes to deactivate the enzyme.

This reaction mixture was treated in the same manner as above and the radioactivity of [³H]guanosine was measured to calculate the enzyme activity.

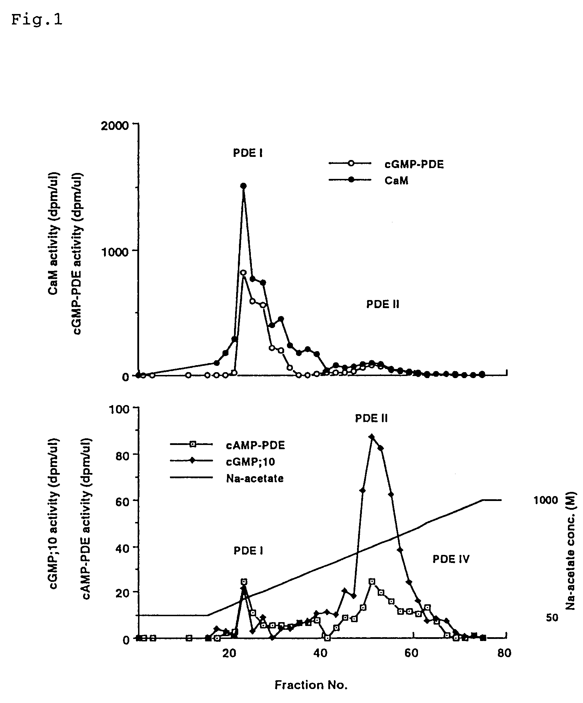

The Mono-Q ion exchange column chromatogram is presented in Fig. 1.

The classification and identification of the isozymes were carried out according to J. A. Beavo et al. (Trends Pharmac. Sci., vol. 11, p. 150, 1990).

Among the fractions possessing cyclic AMP-phosphodiesterase activity, the fraction showing an increase in cyclic GMP-phoshodiesterase activity upon addition of calmodulin was designated PDE I (Fraction I in the diagram).

The fraction showing activation of cyclic AMP-phosphodiesterase activity by cyclic GMP and possessing cyclic GMP-phosphodiesterase activity was designated PDE II (Fraction II in the diagram).

The fraction showing cyclic AMP-phosphodiesterase activity not influenced by addition of calmodulin and cyclic GMP was designated PDE IV (Fraction IV in the diagram).

The separated isozymes were respectively concentrated by means of ultrafiltration membrane (cutoff molecular weight of 5x10⁴) centriflow (Amikon) (final concentrations: PDE I, 2000 µg/ml; PDE II, 200 µg/ml; and PDEIV, 200 µg/ml). To each concentrate were added 10 mM DTT and 0.1% bovine serum albumin and the mixture was stored frozen at -80°C.

The quantitative determination of protein was carried out by the method of Bradford using bovine serum albumin as the standard (Anal. Biochem., Vol. 72, p. 248, 1976). Reference Example 35

### Preparation of PDE isozyme I from the bovine heart

From the bovine heart, PDE isozyme I was prepared in accordance with the methods of R. E. Weishaar et al. (Biochemical Pharmacology, 35, p. 787, 1986) and of B. E. Lavan et al. (Biochemical Pharmacology, 38, p. 4123, 1989).

Using a homogenizer (Nissei Homogenizer AM-12), 30 grams of bovine heart was evenly disrupted in 10 volumes (v/v) of buffer B (10 mM Tris-HCl buffer (pH 7.5) containing 2 mM MgCl₂, 1 mM DTT, 50 µM phenylmethanesulfonyl fluoride (PMSF), and 2 mM benzamidine) in 5 disruption cycles of 10,000 rpm x 30 seconds at 1-minute intervals and, then, centrifuged at 1,000 x g for 10 minutes. The resultant supernatant was filtered through a double wad of gauze, and the precipitate was removed by centrifugation at 100,000 x g for 60 minutes to get a soluble fraction.

To this soluble fraction was added solid ammonium sulfate to 25% saturation and the mixture was stirred for 30 minutes and centrifuged at 10,000 x g for 20 minutes. To the resultant supernatant was added a further amount of solid ammonium sulfate to 65% saturation and after 30 minutes of stirring, the solution was centrifuged at 10,000 x g for 20 minutes. The precipitate obtained was dissolved in 5 ml of buffer C (20 mM Bis-tris-acetate buffer (pH 6.5) containing 5 mM DTT, 2 mM benzamidine, 2 mM EDTA, 50 µM PMSF, and 50 mM sodium acetate). This fraction (containing 100 mg of protein) was dialyzed against 200 volumes of buffer C twice at a 1-hour interval and, then, further dialyzed overnight. After the insoluble fraction was removed by centrifugation (10,000 x g, 20 minutes), the dialyzate was applied to a Mono-Q ion exchange column (Pharmacia, 10 mm x 100 mm) equilibrated with buffer C beforehand. After the column was washed with 60 ml of buffer C, PDE isozymes were eluted on a linear sodium acetate gradient from 0.05 M to 1 M in 240 ml of buffer C. The eluate was collected in 4 ml fractions and PDE activity of each fraction was measured. The above procedure was consistently carried out at 4°C.

The assay, classification, and identification of enzyme activity in the respective fractions were carried out in the same manner as described in Reference Example 34.

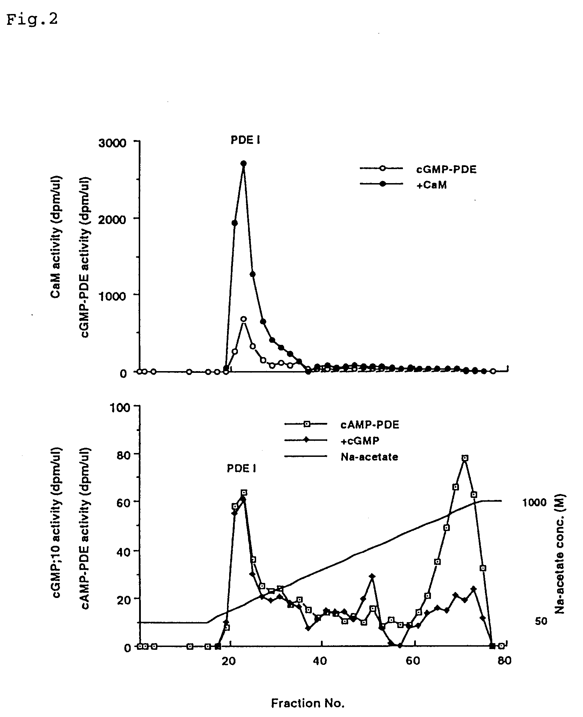

The Mono-Q ion exchange column chromatogram is presented in Fig. 2.

The separated isozyme I was concentrated by means of ultrafiltration membrane (cutoff molecular weight of 5x10⁴) centriflow (Amikon) (final concentration 2000 µg/ml). To the concentrate were added DTT and bovine serum albumin at final concentrations of 10 mM and 0.1%, respectively, and the mixture was stored frozen at -80°C. The assay of protein was carried out by the method of Bradford using bovine serum albumin as the standard (Anal. Biochem., Vol. 72, p. 248, 1976).

### Example 1

### Guanosine 3',5'-cyclic phenylphosphonate

To 2.9 g of N-dimethoxytrityl-2'-O-tetrahydrofuranylguanosine was added 60 ml of a solution of phenyl O,O-bis(1-benzotriazolyl)phosphonate (0.11 M) in dioxane at room temperature. After 30 minutes, 1.81 g of 1-methylimidazole was added and the mixture was stirred at room temperature overnight. To this reaction mixture was added pyridine followed by concentration. The residue was dissolved in methylene chloride and the solution was washed with saturated aqueous sodium chloride solution and concentrated to dryness. The residue was subjected to silica gel column chromatography and elution was carried out with 3% methanol/methylene chloride to provide 2.8 g of cyclic phenyl phosphonate substance. This product was dissolved in 50 ml of dioxane, made acidic (pH 2) with 0.1N hydrochloric acid, and stirred overnight. This reaction mixture was concentrated and the residue was diluted with water. The precipitated white crystals were collected by filtration and washed with ether to provide 700 mg of the title compound. m.p. 230-235°C (decomp.)

### Example 2

### N⁶,N⁶-dimethyladenosine 3',5'-cyclic methylphosphonate

Using the N⁶,N⁶-dimethyl-2'-O-tetrahydrofuranyladenosine obtained in Reference Example 31 and methyl O,O-bis(1-benzotriazolyl)phosphonate, the reaction and acid treatment were carried out in the same manner as Reference Example 1 to provide 0.94 g of the title compound as white crystals. m.p. 230-235°C (decomp.)

| Elemental analysis for C₁₃H₁₈N₅O₅P·3/4H₂O | | | |
|---|---|---|---|
| Calcd. (%): | C, 42.34; | H, 5.33; | N, 18.99 |
| Found (%): | C, 42.34; | H, 5.21; | N, 18.80 |

### Example 3

### 8-(tert-Butylthio)-2'-deoxyadenosine 3',5'-cyclic methylphosphonate

Using the 8-(tert-butylthio)-2'-deoxyadenosine obtained in Reference Example 32 and methyl O,O-bis(1-benzotriazolyl)phosphonate, the procedure of Reference Example 1 was otherwise repeated to provide the title compound. m.p. 220-221°C (decomp.)

| Elemental analysis for C₁₅H₂₂N₅O₄PS·1/4H₂O | | | |
|---|---|---|---|
| Calcd. (%): | C, 44.61; | H, 5.61; | N, 17.34 |
| Found (%): | C, 44.68; | H, 5.49; | N, 17.39 |

### Example 4

### 2'-Deoxy-2'-methylideneadenosine 3',5'-cyclic methylphosphonate

Using the 2'-deoxy-2'-methylideneadenosine obtained in accordance with the literature (Chem. Pharm. Bull., 34, 1518 (1986)) and methyl O,O-bis(1-benzotriazolyl)phosphonate, the procedure of Reference Example 1 was otherwise repeated to provide the title compound. m.p. 144-146°C

| Elemental analysis for C₁₂H₁₄N₅O₄P·4/3H₂O | | | |
|---|---|---|---|
| Calcd. (%): | C, 41.50; | H, 4.84; | N, 20.17 |
| Found (%): | C, 41.64; | H, 4.63; | N, 19.97 |

### Example 5

### 8,2'-S-cycloadenosine 3',5'-cyclic methylphosphonate

Using the 8,2'-S-anhydro(9-β-D-arabinofuranosyl)-8-mercaptoadenosine (alternative name: 8,2'-S-cycloadenosine) obtained in accordance with the literature (Bull. Chem. Soc. Jpn., 48, 3243 (1975)) and methyl O,O-bis(1-benzotriazolyl)phosphonate, the procedure of Reference Example 1 was otherwise repeated to provide the title compound. m.p. 245-252°C (decomp.)

| Elemental analysis for C₁₁H₁₂N₅O₄PS·3/2H₂O | | | |
|---|---|---|---|
| Calcd. (%): | C, 35.87; | H, 4.10; | N, 19.01 |
| Found (%): | C, 36.04; | H, 3.91; | N, 19.32 |

### Example 6

### 8,2'-S-cycloadenosine 3',5'-cyclic propylphosphonate

Using the above 8,2'-S-anhydro(9-β-D-arabinofuranosyl)-8-mercaptoadenosine (alternative name: 8,2'-S-cycloadenosine) and propyl O,O-bis(1-benzotriazolyl)phosphonate, the procedure of Reference Example 1 was otherwise repeated to provide the title compound. m.p. 220-230°C (decomp.)

| Elemental analysis for C₁₃H₁₆N₅O₄PS·H₂O | | | |
|---|---|---|---|
| Calcd. (%): | C, 40.31; | H, 4.68; | N, 18.07 |
| Found (%): | C, 39.95; | H, 4.76; | N, 17.99 |

### Example 7

### 8-Bromo-N⁶,N⁶-dimethyladenosine 3',5'-cyclic methylphosphonate

Using 0.62 g of the N⁶,N⁶-dimethyladenosine 3',5'-cyclic methylphosphonate obtained in Example 2, the procedure of Reference Example 2 was otherwise repeated to provide 0.46 g of the title compound as white powders. m.p. 210-220°C (decomp.)

| Elemental analysis for C₁₃H₁₇BrN₅O₅P·1/2H₂O | | | |
|---|---|---|---|
| Calcd. (%): | C, 35.23; | H, 4.09; | N, 15.80 |
| Found (%): | C, 35.29; | H, 3.91; | N, 15.89 |

### Example 8

### 8-Hydroxyadenosine 3',5'-cyclic methylphosphonate

In 150 ml of acetic acid was dissolved 1.5 g of the 8-bromoadenosine 3',5'-cyclic methylphosphonate obtained in Reference Example 13, followed by addition of 3 g of sodium acetate, and the mixture was stirred at 100°C for 3 hours. The acetic acid was then distilled off and the residue was subjected to reversed phase column chromatography, elution being carried out with 8% methanol-H₂O to provide 0.56 g of the title compound as light-yellow powders.
m.p. 245-250°C (decomp.)

| Elemental analysis for C₁₁H₁₄N₅O₆P·2H₂O | | | |
|---|---|---|---|
| Calcd. (%): | C, 34.93; | H, 4.78; | N, 18.51 |
| Found (%): | C, 34.83; | H, 4.40; | N, 18.22 |

### Example 9

### 8-Hydroxyguanosine 3',5'-cyclic methylphosphonate

Using the 8-bromoguanosine 3',5'-cyclic methylphosphonate obtained in Reference Example 19, the procedure of Example 8 was otherwise repeated to provide the title compound. m.p. 273-277°C (decomp.)

| Elemental analysis for C₁₁H₁₄N₅O₇P·H₂O | | | |
|---|---|---|---|
| Calcd. (%): | C, 35.02; | H, 4.27; | N, 18.56 |
| Found (%): | C, 34.81; | H, 4.12; | N, 18.71 |

### Example 10

### 8-Mercaptoadenosine 3',5'-cyclic methylphosphonate

In 25 ml of ethanol was suspended 500 mg of the 8-bromoadenosine 3',5'-cyclic methylphosphonate obtained in Reference Example 13, followed by addition of 880 mg of thiourea, and the mixture was refluxed for 4 hours. This reaction mixture was concentrated under reduced pressure and the residue was diluted with water. The precipitated crystals were collected by filtration, washed with water, and dried to provide 152.9 mg of the title compound as light-brown crystals. m.p. 220-230°C (decomp.)

| Elemental analysis for C₁₁H₁₄N₅O₅PS·H₂O | | | |
|---|---|---|---|
| Calcd. (%): | C, 35.02; | H, 4.27; | N, 18.56 |
| Found (%): | C, 35.27; | H, 4.10; | N, 18.88 |

### Example 11

### 8-Mercaptoadenosine 3',5'-cyclic ethylphosphonate

Using the 8-bromoadenosine 3',5'-cyclic ethylphosphonate obtained in Reference Example 14, the procedure of Example 10 was otherwise repeated to provide the title compound. m.p. 230-240°C (decomp.)

| Elemental analysis for C₁₂H₁₆N₅O₅PS·1/2H₂O | | | |
|---|---|---|---|
| Calcd. (%): | C, 37.70; | H, 4.48; | N, 18.32 |
| Found (%): | C, 37.55; | H, 4.35; | N, 18.52 |

### Example 12

### 8-Mercaptoguanosine 3',5'-cyclic methylphosphonate

Using the 8-bromoguanosine 3',5'-cyclic methylphosphonate obtained in Reference Example 19, the procedure of Example 10 was otherwise repeated to provide the title compound. m.p. 226-238°C (decomp.)

| Elemental analysis for C₁₁H₁₄N₅O₆PS·H₂O | | | |
|---|---|---|---|
| Calcd. (%): | C, 33.59; | H, 4.10; | N, 17.81 |
| Found (%): | C, 33.74; | H, 4.01; | N, 18.13 |

### Example 13

### 2'-Deoxy-8-mercaptoguanosine 3',5'-cyclic methylphosphonate

Using the 8-bromo-2'-deoxyguanosine 3',5'-cyclic methylphosphonate obtained in Reference Example 22, the procedure of Example 10 was otherwise repeated to provide the title compound.
m.p. 228-232°C (decomp.)

| Elemental analysis for C₁₁H₁₄N₅O₅PS·3/2H₂O | | | |
|---|---|---|---|
| Calcd. (%): | C, 34.20; | H, 4.44; | N, 18.12 |
| Found (%): | C, 34.43; | H, 4.38; | N, 17.86 |

### Example 14

### 2'-Deoxy-8-mercaptoadenosine 3',5'-cyclic methylphosphonate

Using the 8-bromo-2'-deoxyadenosine 3',5'-cyclic methylphosphonate obtained in Reference Example 16, the procedure of Example 10 was otherwise repeated to provide the title compound.
m.p. 140-145°C, 250°C (decomp.)

### Example 15

### 2'-O-acetyl-8-mercaptoguanosine 3',5'-cyclic methylphosphonate

Using the 2'-O-acetyl-8-bromoguanosine 3',5'-cyclic methylphosphonate obtained in Reference Example 28, the procedure of Example 10 was otherwise repeated to provide the title compound.
m.p. 225-230°C (decomp.)

| Elemental analysis for C₁₃H₁₆N₅O₇PS·2H₂O | | | |
|---|---|---|---|
| Calcd. (%): | C, 34.44; | H, 4.45; | N, 15.45 |
| Found (%): | C, 34.67; | H, 4.42; | N, 15.37 |

### Example 16

### 2'-O-propionyl-8-mercaptoguanosine 3',5'-cyclic propylphosphonate

Using the 2'-O-propionyl-8-bromoguanosine 3',5'-cyclic propylphosphonate obtained in Reference Example 30, the procedure of Example 10 was otherwise repeated to provide the title compound.
m.p. 235-240°C (decomp.)

### Example 17

### 8-Methylthioadenosine 3',5'-cyclic methylphosphonate

In 5 ml of DMF was dissolved 100 mg of the 8-mercaptoadenosine 3',5'-cyclic methylphosphonate obtained in Example 10, followed by addition of 58 mg of potassium carbonate and 48 mg of methyl iodide and then the mixture was stirred for 1 hour. This reaction mixture was made acidic with 50% aqueous acetic acid solution and concentrated under reduced pressure. The residue was subjected to reversed phase column chromatography and elution was carried out with methanol-water (10%-30% gradient) to provide 89 mg of the title compound as white powders. m.p. 220-230°C (decomp.)

| Elemental analysis for C₁₂H₁₆N₅O₅PS | | | |
|---|---|---|---|
| Calcd. (%): | C, 38.61; | H, 4.32; | N, 18.54 |
| Found (%): | C, 38.39; | H, 4.24; | N, 18.76 |

### Example 18

### 8-Methylthioadenosine 3',5'-cyclic ethylphosphonate

Using the 8-mercaptoadenosine 3',5'-cyclic ethylphosphate obtained in Example 11 and ethyl iodide, the procedure of Example 17 was otherwise repeated to provide the title compound. m.p. 230-237°C (decomp.)

| Elemental analysis for C₁₃H₁₈N₅O₅PS | | | |
|---|---|---|---|
| Calcd. (%): | C, 40.31; | H, 4.68; | N, 18.08 |
| Found (%): | C, 40.49; | H, 4.60; | N, 17.86 |

### Example 19

### 8-Ethylthioadenosine 3',5'-cyclic methylphosphonate

Using the 8-mercaptoadenosine 3',5'-cyclic methylphosphonate obtained in Example 10 and ethyl iodide, the procedure of Example 17 was otherwise repeated to provide the title compound. m.p. 215-220°C (decomp.)

| Elemental analysis for C₁₃H₁₈N₅O₅PS | | | |
|---|---|---|---|
| Calcd. (%): | C, 40.31; | H, 4.68; | N, 18.08 |
| Found (%): | C, 40.38; | H, 4.64; | N, 17.76 |

### Example 20

### 8-Propylthioadenosine 3',5'-cyclic methylphosphonate

Using the 8-mercaptoadenosine 3',5'-cyclic methylphosphonate obtained in Example 10 and propyl iodide, the procedure of Example 17 was otherwise repeated to provide the title compound. m.p. 220-225°C (decomp.)

| Elemental analysis for C₁₄H₂₀N₅O₅PS | | | |
|---|---|---|---|
| Calcd. (%): | C, 41.89; | H, 5.02; | N, 17.45 |
| Found (%): | C, 41.91; | H, 4.86; | N, 17.20 |

### Example 21

### 8-Propylthioadenosine 3',5'-cyclic ethylphosphonate

Using the 8-mercaptoadenosine 3',5'-cyclic ethylphosphonate obtained in Example 11, the procedure of Example 17 was otherwise repeated to provide the title compound. m.p. 130-135°C (decomp.)

### Example 22

### 8-Propylthioadenosine 3',5'-cyclic propylphosphonate

Using the 8-bromoadenosine 3',5'-cyclic propylphosphonate obtained in Reference Example 15, the procedure of Example 10 was otherwise repeated to provide 8-mercaptoadenosine 3',5'-cyclic propylphosphonate. Using this compound and propyl iodide, the procedure of Example 17 was otherwise repeated to provide the title compound. m.p. 192-193°C

| Elemental analysis for C₁₆H₂₄N₅O₅PS | | | |
|---|---|---|---|
| Calcd. (%): | C, 44.75; | H, 5.63; | N, 16.31 |
| Found (%): | C, 44.42; | H, 5.60: | N, 16.01 |

### Example 23

### 8-Isopropylthioadenosine 3',5'-cyclic ethylphosphonate

Using the 8-mercaptoadenosine 3',5'-cyclic ethylphosphonate obtained in Example 11 and isopropyl iodide, the procedure of Example 17 was otherwise repeated to provide the title compound.
m.p. 205-208°C (decomp.)

| Elemental analysis for C₁₅H₂₂N₅O₅PS | | | |
|---|---|---|---|
| Calcd. (%): | C, 43.37; | H, 5.34; | N, 16.86 |
| Found (%): | C, 43.43; | H, 5.48; | N, 16.72 |

### Example 24

### 8-Cyclopentylthioadenosine 3',5'-cyclic propylphosphonate

Using 8-mercaptoadenosine 3',5'-cyclic propylphosphonate and cyclopentyl bromide, the procedure of Example 17 was otherwise repeated to provide the title compound. m.p. 120-128°C

| Elemental analysis for C₁₈H₂₆N₅O₅PS·1/2H₂O | | | |
|---|---|---|---|
| Calcd. (%): | C, 46.54; | H, 5.86; | N, 15.08 |
| Found (%): | C, 46.68; | H, 5.88; | N, 14.64 |

### Example 25

### 8-[2-(2-Methoxyethoxy)ethylthio]adenosine 3',5'-cyclic propylphosphonate

Using 8-mercaptoadenosine 3',5'-cyclic propylphosphonate and 2-(2-methoxyethoxy)ethyl bromide, the procedure of Example 17 was otherwise repeated to provide the title compound. m.p. 200°C (decomp.)

| Elemental analysis for C₁₈H₂₈N₅O₇PS·H₂O | | | |
|---|---|---|---|
| Calcd. (%): | C, 42.60; | H, 5.96; | N, 13.80 |
| Found (%): | C, 42.82; | H, 5.62; | N, 13.55 |

### Example 26

### 2'-Deoxy-8-methylthioadenosine 3',5'-cyclic methylphosphonate

Using the 2'-deoxy-8-mercaptoadenosine 3',5'-cyclic methylphosphonate obtained in Example 14 and methyl iodide, the procedure of Example 17 was otherwise repeated to provide the title compound.
m.p. 215-218°C (decomp.)

| Elemental analysis for C₁₂H₁₆N₅O₄PS | | | |
|---|---|---|---|
| Calcd. (%): | C, 40.34; | H, 4.51; | N, 19.60 |
| Found (%): | C, 40.59; | H, 4.51; | N, 19.18 |

### Example 27

### 2'-Deoxy-8-hexylthioadenosine 3',5'-cyclic propylphosphonate

Using the 8-bromo-2'-deoxyadenosine 3',5'-cyclic propylphosphonate obtained in Reference Example 18, the procedure of Example 10 was otherwise repeated to provide 2'-deoxy-8-mercaptoadenosine 3',5'-cyclic propylphosphonate. Then, using this compound and hexyl bromide, the title compound was synthesized in the same manner as Example 17. m.p. 208-209°C

| Elemental analysis for C₁₉H₃₀N₅O₄PS | | | |
|---|---|---|---|
| Calcd. (%): | C, 50.10; | H, 6.64; | N, 15.37 |
| Found (%): | C, 49.68; | H, 6.65; | N, 15.33 |

### Example 28

### 2'-Deoxy-8-(2-hydroxyethylthio)adenosine 3',5'-cyclic ethylphosphonate

Using the 8-bromo-2'-deoxyadenosine 3',5'-cyclic ethylphosphonate obtained in Reference Example 17, the procedure of Example 10 was otherwise repeated to provide 2'-deoxy-8-mercaptoadenosine 3',5'-cyclic ethylphosphonate. Then, using this compound and 2-hydroxyethyl bromide, the title compound was synthesized in the same manner as Example 17.
m.p. 208-214°C (decomp.)

| Elemental analysis for C₁₄H₂₀N₅O₅PS | | | |
|---|---|---|---|
| Calcd. (%): | C, 41.89; | H, 5.02; | N, 17.45 |
| Found (%): | C, 41.84; | H, 4.98; | N, 17.22 |

### Example 29

### 8-Methylthioguanosine 3',5'-cyclic methylphosphonate

Using the 8-mercaptoguanosine 3',5'-cyclic methylphosphonate obtained in Example 12 and methyl iodide, the procedure of Example 17 was otherwise repeated to provide the title compound. m.p. 223-237°C (decomp.)

| Elemental analysis for C₁₂H₁₆N₅O₆PS·1/2H₂O | | | |
|---|---|---|---|
| Calcd. (%): | C, 36.18; | H, 4.30; | N, 17.58 |
| Found (%): | C, 36.90; | H, 4.01; | N, 17.04 |

### Example 30

### 8-[2-(2-Methoxyethoxy)ethylthio]guanosine 3',5'-cyclic methylphosphonate

Using the 8-mercaptoguanosine 3',5'-cyclic methylphosphonate obtained in Example 12 and 2-(2-methoxyethoxy)ethyl bromide, the procedure of Example 17 was otherwise repeated to provide the title compound.
m.p. 155-204°C (decomp.)

| Elemental analysis for C₁₆H₂₄N₅O₈PS·1/2H₂O | | | |
|---|---|---|---|
| Calcd. (%): | C, 39.51; | H, 5.18; | N, 14.40 |
| Found (%): | C, 39.78; | H, 5.22; | N, 14.03 |

### Example 31

### 8-(Carboxymethylthio)guanosine 3',5'-cyclic methylphosphonate

Using the 8-mercaptoguanosine 3',5'-cyclic methylphosphonate obtained in Example 12 and carboxymethyl bromide, the procedure of Example 17 was otherwise repeated to provide the title compound.
m.p. 213-236°C (decomp.)

| Elemental analysis for C₁₃H₁₆N₅O₈PS·1/2H₂O | | | |
|---|---|---|---|
| Calcd. (%): | C, 35.29; | H, 3.87; | N, 15.83 |
| Found (%): | C, 35.04; | H, 4.00; | N, 15.12 |

### Example 32

### 2'-Deoxy-8-methylthioguanosine 3',5'-cyclic methylphosphonate

Using the 2'-deoxy-8-mercaptoguanosine 3',5'-cyclic methylphosphonate obtained in Example 13 and methyl iodide, the procedure of Example 17 was otherwise repeated to provide the title compound.
m.p. 210-228°C (decomp.)

| Elemental analysis for C₁₂H₁₆N₅O₅PS | | | |
|---|---|---|---|
| Calcd. (%): | C, 38.61; | H, 4.32; | N, 18.76 |
| Found (%): | C, 38.84; | H, 4.14; | N, 18.56 |

### Example 33

### 2'-Deoxy-8-(2-hydroxyethylthio)guanosine 3',5'-cyclic ethylphosphonate

Using the 8-bromo-2'-deoxyguanosine 3',5'-cyclic ethylphosphonate obtained in Reference Example 23, the procedure of Example 10 was otherwise repeated to provide 2'-deoxy-8-mercaptoguanosine 3',5'-cyclic ethylphosphonate. Then, using this compound and 2-hydroxyethyl bromide, the title compound was synthesized in the same manner as Example 17.
m.p. 186-193°C (decomp.)

| Elemental analysis for C₁₄H₂₀N₅O₆PS·1/2H₂O | | | |
|---|---|---|---|
| Calcd. (%): | C, 39.44, | H, 4.96; | N, 16.42 |
| Found (%): | C, 39.03; | H, 4.94; | N, 16.31 |

### Example 34

### 2'-Deoxy-8-(2-hydroxyethylthio)guanosine 3',5'-cyclic propylphosphonate

Using the 8-bromo-2'-deoxyguanosine 3',5'-cyclic propylphosphonate obtained in Reference Example 24, the procedure of Example 10 was otherwise repeated to provide 2'-deoxy-8-mercaptoguanosine 3',5'-cyclic propylphosphonate. Then, using this compound and 2-hydroxyethyl bromide, the title compound was synthesized in the same manner as Example 17.
m.p. 165-172°C (decomp.)

| Elemental analysis for C₁₅H₂₂N₅O₆PS·H₂O | | | |
|---|---|---|---|
| Calcd. (%): | C, 40.09, | H, 5.38; | N, 15.58 |
| Found (%): | C, 40.33, | H, 5.18; | N, 15.28 |

### Example 35

### 2'-O-acetyl-8-(2-hydroxyethylthio)guanosine 3',5'-cyclic methylphosphonate

Using the 2'-O-acetyl-8-mercaptoguanosine 3',5'-cyclic methylphosphonate obtained in Example 15 and 2-hydroxyethyl bromide, the procedure of Example 17 was otherwise repeated to provide the title compound.
m.p. 255-260°C (decomp.)

| Elemental analysis for C₁₅H₂₀N₅O₈PS | | | |
|---|---|---|---|
| Calcd. (%): | C, 39.05, | H, 4.37; | N, 15.18 |
| Found (%): | C, 38.76, | H, 4.19; | N, 15.03 |

### Example 36

### 2'-O-acetyl-8-(2-hydroxyethylthio)guanosine 3',5'-cyclic propylphosphonate

Using the 2'-O-acetyl-8-bromoguanosine 3',5'-cyclic propylphosphonate obtained in Reference Example 29, the procedure of Example 10 was otherwise repeated to provide 2'-O-acetyl-8-mercaptoguanosine 3',5'-cyclic propylphosphonate. Then, using this compound and 2-hydroxyethyl bromide, the title compound was synthesized in the same manner as Example 17.
m.p. 242-247°C (decomp.)

### Example 37

### 2'-O-acetyl-8-(3-hydroxypropylthio)guanosine 3',5'-cyclic methylphosphonate

Using 2'-O-acetyl-8-mercaptoguanosine 3',5'-cyclic propylphosphonate and 3-hydroxypropyl bromide, the procedure of Example 17 was otherwise repeated to provide the title compound.
m.p. 240-243°C (decomp.)

| Elemental analysis for C₁₆H₂₂N₅O₈PS | | | |
|---|---|---|---|
| Calcd. (%): | C, 40.42, | H, 4.66; | N, 14.73 |
| Found (%): | C, 40.37, | H, 4.84; | N, 14.21 |

### Example 38

### 8-(2-Hydroxyethylthio)-2'-O-propionylguanosine 3',5'-cyclic propylphosphonate

Using the 2'-O-propionyl-8-mercaptoguanosine 3',5'-cyclic propylphosphonate obtained in Example 16 and 2-hydroxyethyl bromide, the procedure of Example 17 was otherwise repeated to provide the title compound. m.p. 165-168°C

| Elemental analysis for C₁₈H₂₆N₅O₈PS·1/2H₂O | | | |
|---|---|---|---|
| Calcd. (%): | C, 42.19, | H, 5.31; | N, 13.67 |
| Found (%): | C, 42.33, | H, 5.25; | N, 13.25 |

### Example 39

### 8-(4-Chlorophenylthio)adenosine 3',5'-cyclic methylphosphonate

In 10 ml of DMF was dissolved 300.2 mg of 8-bromoadenosine 3',5'-cyclic methylphosphonate, followed by addition of 200.4 mg of potassium carbonate and 0.16 g of 4-chlorothiophenol, and the mixture was stirred for 2 hours. This reaction mixture was poured in acetic acid-water and extracted with ethyl acetate thrice. The organic layer was taken and dried over anhydrous magnesium sulfate and the solvent was distilled off. The residue was subjected to silica gel column chromatography and elution was carried out with 10% methanol-methylene chloride to provide 340 mg of the title compound as white powders. m.p. 150-165°C

| Elemental analysis for C₁₇H₁₇ClN₅O₅PS·1/2H₂O | | | |
|---|---|---|---|
| Calcd. (%): | C, 42.64, | H, 3.79; | N, 14.63 |
| Found (%): | C, 42.47, | H, 3.65; | N, 14.81 |

### Example 40

### 2'-O-methanesulfonyladenosine 3',5'-cyclic methylphosphonate

In a mixture of methylene chloride (10 ml) and pyridine (10 ml) was dissolved 0.5 g of adenosine 3',5'-cyclic methyl phosphonate and 0.5 g of 4-dimethylaminopyridine. To this solution was added a solution of 1 g methanesulfonyl chloride in methylene chloride dropwise and the mixture was stirred for 4 hours. This reaction mixture was concentrated and the residue was subjected to reversed phase column chromatography. Elution was carried out with 40% methanol-water to provide 0.54 g of the title compound as white crystals. m.p. 146-148°C

### Example 41

### 8-Bromo-2'-O-(p-toluenesulfonyl)adenosine 3',5'-cyclic methylphosphonate

In 5 ml of pyridine was dissolved 0.15 g of the 8-bromoadenosine 3',5'-cyclic methylphosphonate obtained in Reference Example 13, followed by addition of 0.24 g of 1-methylimidazole and 0.22 g of p-toluenesulfonyl chloride, and the mixture was stirred for 2 hours. This reaction mixture was concentrated and purified by preparative silica gel TLC to provide 0.16 g of light-brown solid.
¹H-NMR (CDCl₃) δ: 1.62 (3H, d, J=18 Hz), 2.44 (3H, s), 4.05-4.25 (1H, m), 4.35-4.6 (2H, m), 4.7 (2H, bs), 5.5-5.7 (1H, m), 5.81 (1H, d, J=8 Hz), 6.13 (1H, s), 7.28 (2H, d, J=10 Hz), 7.7 (2H, d, J=10 Hz), 8.25 (1H, s)

### Example 42

### 8-Bromo-2'-O-trifluoromethanesulfonyladenosine 3',5'-cyclic methylphosphonate

In a mixture of methylene chloride (30 ml) and pyridine (10 ml) were dissolved 430 mg of the 8-bromoadenosine 3',5'-cyclic methylphosphonate obtained in Reference Example 13 and 660 mg of 1-methylimidazole. Then, 1.13 g of trifluoromethanesulfonic anhydride was added dropwise under ice-cooling and the mixture was stirred for 30 minutes. This reaction mixture was poured in ice-water. The organic layer was separated and then washed with 0.2N aqueous monopotassium phosphate and dried over anhydrous magnesium sulfate. The solvent was then distilled off and the residue was subjected to silica gel column chromatography using 5% methanolmethylene chloride as eluent to provide 430 mg of the title compound as light-yellow solid.
UV λmax (MeOH) = 263.6, 212.6

### Example 43

### 2'-Deoxy-8,2'-β-dibromoadenosine 3',5'-cyclic methylphosphonate

In 10 ml of DMF was dissolved 280 mg of the 8-bromo-2'-O-trifluoromethanesulfonyladenosine 3',5'-cyclic methylphosphonate obtained in Example 42 followed by addition of 540 mg of lithium bromide. This mixture was stirred overnight and then warmed at 40 °C for 1 hour. After addition of acetic acid, the reaction mixture was concentrated. The residue was subjected to reversed phase column chromatography using 30% methanol-H₂O as eluent to provide 128.1 mg of the title compound as white powders. m.p. 218-220°C (decomp.)

| Elemental analysis for C₁₁H₁₂Br₂N₅O₄P | | | |
|---|---|---|---|
| Calcd. (%): | C, 28.17, | H, 2.58; | N, 14.93 |
| Found (%): | C, 28.13, | H, 2.42; | N, 15.02 |

### Example 44

### 2'-Deoxy-2'-β-bromoadenosine 3',5'-cyclic methylphosphonate

In 20 ml of methanol was dissolved 76.5 mg of the 2'-deoxy-8,2'-β-dibromoadenosine 3',5'-cyclic methylphosphonate obtained in Example 43, followed by addition of 20 mg of 5% palladium-on-carbon and then a hydrogenation reaction was carried out at atmospheric pressure for 5 hours. The catalyst was then filtered off and the filtrate was concentrated. The residue was subjected to reversed phase column chromatography and elution was carried out with methanol-water (10%-20% gradient) to provide 39.5 mg of the title compound as white powders. m.p. 215-220°C (decomp.)
FAB-MS: 390 (M+H)⁺

### Example 45

### 2'-O-trifluoromethanesulfonyladenosine 3',5'-cyclic methylphosphonate hydrobromide

In 50 ml of methanol was dissolved 740 mg of the 8-bromo-2'-O-trifluoromethanesulfonyladenosine 3',5'-cyclic methylphosphonate obtained in Example 42, followed by addition of 0.1 g of 5% palladium-on-carbon, and a hydrogenation reaction was carried out at atmospheric pressure for 2 hours. The catalyst was then filtered off and the filtrate was concentrated to provide 670 mg of the title compound as white solid.
m.p. 138-140°C (decomp.)

### Example 46

### 2'-Deoxy-2'-β-chloroadenosine 3',5'-cyclic methylphosphonate

In 20 ml of DMF was dissolved 380 mg of 2'-O-trifluoromethanesulfonyladenosine 3',5'-cyclic methylphosphonate hydrobromide obtained in Example 45, followed by addition of 150 mg of lithium chloride, and the mixture was stirred for 6 hours. After addition of acetic acid, the reaction mixture was concentrated and the residue was subjected to reversed phase column chromatography using 20% methanol-H₂O as eluent to provide 127.5 mg of the title compound as white powders. m.p. 230-235°C (decomp.)

| Elemental analysis for C₁₁H₁₃ClN₅O₄P·1/4H₂O | | | |
|---|---|---|---|
| Calcd. (%): | C, 37.73, | H, 3.89; | N, 20.00 |
| Found (%): | C, 37.50, | H, 3.82; | N, 20.07 |

### Example 47

### 2'-Deoxy-2'-β-chloroadenosine 3',5'-cyclic propylphosphonate

Using the 8-bromoadenosine 3',5'-cyclic propylphosphonate obtained in Reference Example 15, the procedure of Example 42 was otherwise repeated to synthesize 8-bromo-2'-O-trifluoromethanesulfonyladenosine 3',5'-cyclic propylphosphonate. Using this compound, the procedure of Example 45 was otherwise repeated to provide 2'-O-trifluoromethanesulfonyladenosine 3',5'-cyclic propylphosphonate hydrobromide. Using this compound, the title compound was produced in otherwise the same manner as

### Example 46.

m.p. 210-220°C (decomp.)
FAB-MS: 374 (M+H)⁺

### Example 48

### N⁶,N⁶-dimethyl-2'-deoxy-2'-β-chloroadenosine 3',5'-cyclic methylphosphonate

Using the 8-bromo-N⁶,N⁶-dimethyladenosine 3',5'-cyclic methylphosphonate obtained in Example 7, the procedure of Example 42 was otherwise repeated to provide 8-bromo-N⁶,N⁶-dimethyl-2'-O-trifluoromethanesulfonyladenosine 3',5'-cyclic methylphosphonate. Using this compound, the procedure of Example 45 was otherwise repeated to provide N⁶,N⁶-dimethyl-2'-O-trifluoromethanesulfonyladenosine 3',5'-cyclic methylphosphonate hydrobromide. Starting with this compound, the title compound was synthesized in the same manner as Example 46. m.p. 145-150°C

| Elemental analysis for C₁₃H₁₇ClN₅O₄P | | | |
|---|---|---|---|
| Calcd. (%): | C, 41.78, | H, 4.58; | N, 18.74 |
| Found (%): | C, 41.55, | H, 4.40; | N, 18.68 |

### Example 49

### 2'-Deoxy-2'-β-methyladenosine 3',5'-cyclic methylphosphonate

In 50 ml of 2% acetic acid-methanol was dissolved 126.3 mg of the 2'-deoxy-2'-methylideneadenosine 3',5'-cyclic methylphosphonate obtained in Example 4, followed by addition of 65 mg of 5% palladium-on-carbon and a hydrogenation reaction was carried out at 3 atmospheres overnight. The catalyst was then filtered off, the filtrate was concentrated, and the residue was subjected to reversed phase column chromatography. Elution was carried out with 20% methanol-H₂O to provide 74.5 mg of the title compound as white powders. m.p. 205-210°C (decomp.)

| Elemental analysis for C₁₂H₁₆N₅O₄P·H₂O | | | |
|---|---|---|---|
| Calcd. (%): | C, 41.99, | H, 5.29; | N, 20.40 |
| Found (%): | C, 41.77, | H, 5.40; | N, 20.21 |

### Example 50

### 8-Methylguanosine 3',5'-cyclic methylphosphonate

In 60 ml of 0.3N sulfuric acid was dissolved 300 mg of the guanosine 3',5'-cyclic methylphosphonate obtained in Reference Example 7. Then, under ice-cooling, 6 ml of an aqueous solution of ferrous sulfate heptahydrate (1.46 g) and 6 ml of an aqueous solution of tert-butyl hydroperoxide (315 mg) were concurrently added and the mixture was stirred for 3.5 hours. Then, 2 ml of an aqueous solution of tert-butyl hydroperoxide (100 mg) was further added and the mixture was stirred for 1 hour. The reaction mixture was then concentrated under reduced pressure. The residue was subjected to reversed phase column chromatography using methanol-water (0-20% gradient) as eluent to provide 160 mg of the title compound as white powders.
m.p. 210-228°C (decomp.)

| Elemental analysis for C₁₂H₁₆N₅O₆P·3/2H₂O | | | |
|---|---|---|---|
| Calcd. (%): | C, 37.51, | H, 4.98; | N, 18.22 |
| Found (%): | C, 37.47, | H, 4.53; | N, 18.25 |

### Example 51

### 8-Methylguanosine 3',5'-cyclic ethylphosphonate

Using the guanosine 3',5'-cyclic ethylphosphonate obtained in Reference Example 8, the procedure of Example 50 was otherwise repeated to provide the title compound. m.p. 244-253°C

| Elemental analysis for C₁₃H₁₈N₅O₆P·H₂O | | | |
|---|---|---|---|
| Calcd. (%): | C, 40.01, | H, 5.18; | N, 17.99 |
| Found (%): | C, 39.96, | H, 5.30; | N, 17.06 |

### Example 52

### 8-Methylguanosine 3',5'-cyclic propylphosphonate

Using the guanosine 3',5'-cyclic propylphosphonate obtained in Reference Example 9, the procedure of Example 50 was otherwise repeated to provide the title compound. m.p. 242-273°C (decomp.)

| Elemental analysis for C₁₄H₂₀N₅O₆P | | | |
|---|---|---|---|
| Calcd. (%): | C, 43.64, | H, 5.23; | N, 18.18 |
| Found (%): | C, 43.35, | H, 5.03; | N, 17.80 |

### Example 53

### 2'-Deoxy-8-methylguanosine 3',5'-cyclic methylphosphonate

Using the 2'-deoxyguanosine 3',5'-cyclic methylphosphonate obtained in Reference Example 10, the procedure of Example 50 was otherwise repeated to provide the title compound.
m.p. 236-242°C (decomp.)

| Elemental analysis for C₁₂H₁₆N₅O₅P·1/2H₂O | | | |
|---|---|---|---|
| Calcd. (%): | C, 41.15, | H, 4.89; | N, 19.99 |
| Found (%): | C, 41.05, | H, 4.72, | N, 19.41 |

### Example 54

### 2'-Deoxy-8-methylguanosine 3',5'-cyclic ethylphosphonate

Using the 2'-deoxyguanosine 3',5'-cyclic ethylphosphonate obtained in Reference Example 11, the procedure of Example 50 was otherwise repeated to provide the title compound.
m.p. 242-250°C (decomp.)

| Elemental analysis for C₁₃H₁₈N₅O₅P·1/2H₂O | | | |
|---|---|---|---|
| Calcd. (%): | C, 42.86, | H, 5.26; | N, 19.22 |
| Found (%): | C, 42.18, | H, 5.32, | N, 19.41 |

### Example 55

### Xanthosine 3',5-cyclic methylphosphate

In 10 ml of 2N acetic acid was suspended 100 mg of the guanosine 3',5-cyclic methylphosphate obtained in Reference Example 7, followed by addition of 6 ml of aqueous sodium nitrite solution (2 g/7.5 ml), and the mixture was stirred for 4.5 hours. This reaction mixture was concentrated and the residue was subjected to reversed phase column chromatography using 10% methanol-water as eluent to provide 50 mg of the title compound as white powders. m.p. 233-242°C (decomp.)

| Elemental analysis for C₁₁H₁₃N₄O₇P·H₂O | | | |
|---|---|---|---|
| Calcd. (%): | C, 36.47, | H, 4.17; | N, 15.47 |
| Found (%): | C, 36.58, | H, 4.06, | N, 15.19 |

The compounds produced in the above-mentioned examples are shown in Table 2.

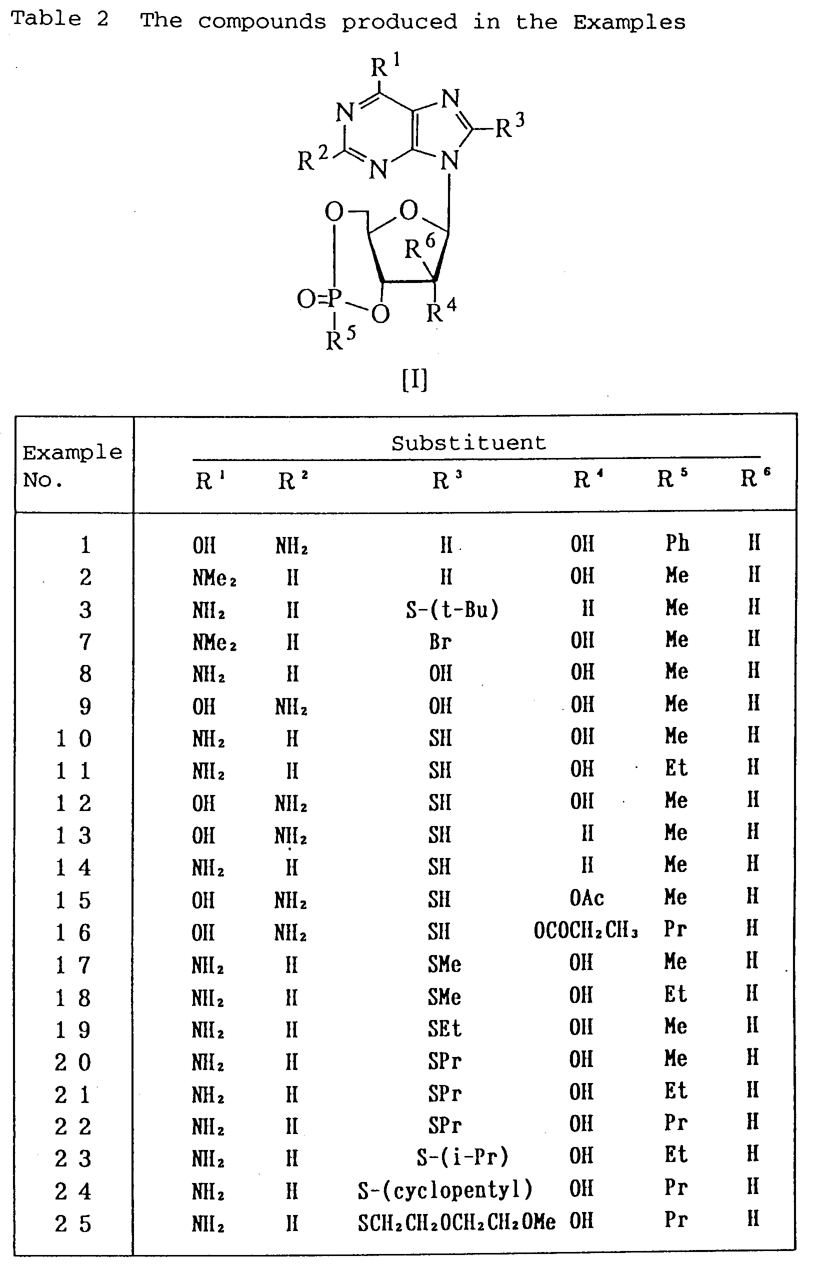

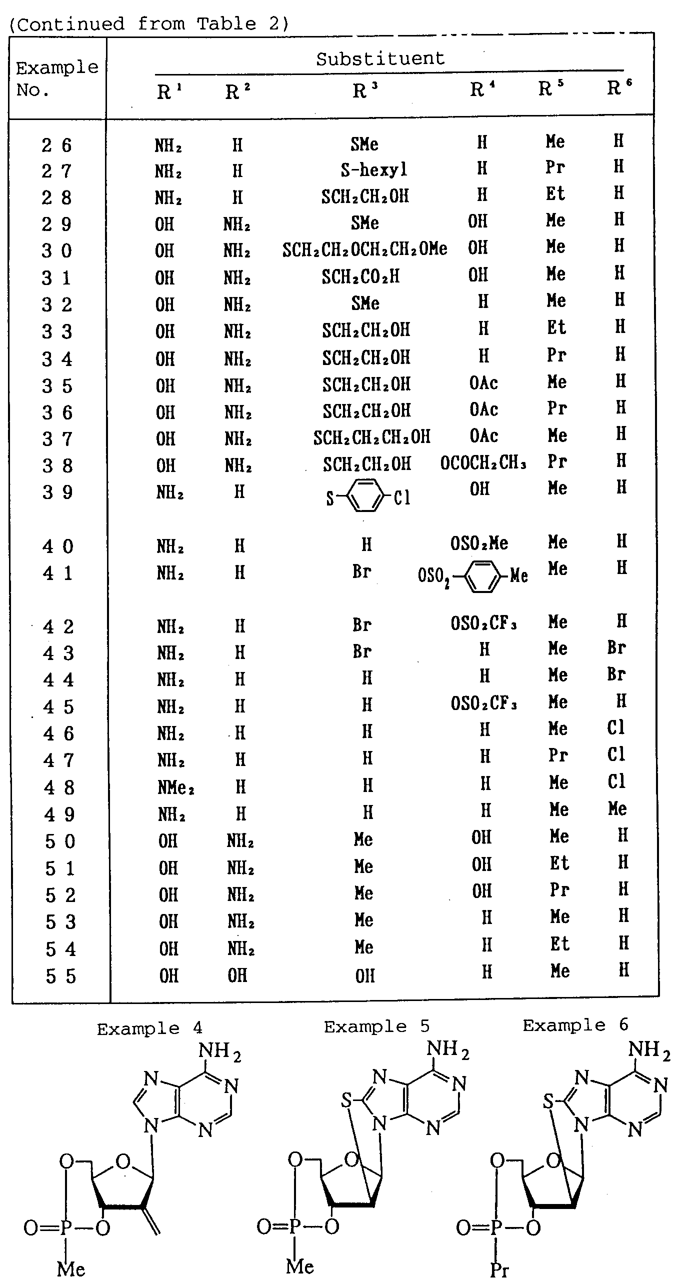

### BRIEF DESCRIPTION OF THE DRAWINGS

Fig. 1 show Mono-Q ion exchange chromatograms of bovine bladder-derived PDE isozymes I, II, and IV.

The abscissa represents fraction No. and the ordinate represents enzyme activity. Top: cyclic GMP-phosphodiesterase activity (cGMP-PDE) and calcium/calmodulin-dependent cyclic GMP-phosphodiesterase activity (CaM); bottom: cyclic AMP-phosphodiesterase activity (cAMP-PDE) and cyclic GMP-stimulating cyclic AMP-phosphodiesterase activity (cGMP; 10). Na-acetate represents the concentration gradient of sodium acetate.

In the diagram, PDE I, PDE II, and PDE IV represents PDE isozyme I active fraction, PDE isozyme II active fraction, and PDE isozyme IV active fraction, respectively.

Fig. 2 is a Mono-Q ion exchange chromatogram of bovine heart-derived PDE isozyme I.

The abscissa represents fraction No. and the ordinate represents enzyme activity. Top: cyclic GMP-phosphodiesterase activity (cGMP-PDE) and calcium/calmodulin-dependent cyclic GMP-phosphodiesterase activity (CaM); bottom: cyclic AMP-phosphodiesterase activity (cAMP-PDE) and cyclic GMP-stimulating cyclic AMP-phosphodiesterase activity (cGMP; 10). Na-acetate represents the concentration gradient of sodium acetate.

In the diagram, PDE I represents PDE isozyme I active fraction.

## Claims

1. A compound of the following formula [I] or a pharmaceutically acceptable salt thereof. 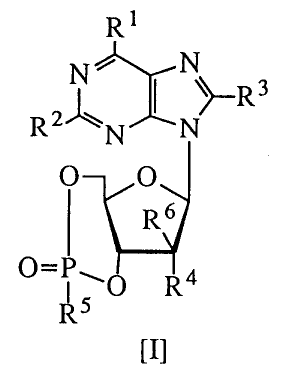 wherein
R¹ represents hydroxy, amino, or -NR¹¹R¹² where R¹¹ and R¹² may be the same or different and each represents alkyl.
R² represents hydrogen, amino, or hydroxy.
R³ represents hydrogen, halogen, alkyl, hydroxy, mercapto, or -S-X where X represents alkyl which is either unsubstituted or substituted (the substituent is selected from among hydroxy, carboxy, and alkoxyalkoxy), cycloalkyl, or aryl which is either unsubstituted or substituted by halogen.
R⁴ represents hydrogen, hydroxy, acyloxy, alkoxy, or -OSO₂-Y in which Y represents aryl which is either unsubstituted or substituted by alkyl, or alkyl which is either unsubstituted or substituted by halogen.
R⁵ represents alkyl or aryl.
R⁶ represents hydrogen, halogen, or alkyl.
R⁴ and R⁶ may jointly represent =CH₂.
R³ and R⁶ may jointly represent -S-.
In the case that R⁴ and R⁶ are independent of each other, at least either one represents hydrogen.
Excluded are the case in which R¹ represents amino; R² represents hydrogen; R³ represents hydrogen or halogen; R⁴ represents hydrogen, hydroxy, or acyloxy; R⁵ represents alkyl; R⁶ represents hydrogen, the case in which R¹ represents hydroxy; R² represents hydrogen; R³ represents hydrogen, halogen, alkyl, or hydroxy; R⁴ represents hydrogen, hydroxy, acyloxy, or alkoxy; R⁵ represents alkyl; R⁶ represents hydrogen, and the case in which R¹ represents hydroxy; R² represents amino; R³ represents hydrogen or halogen; R⁴ represents hydrogen, hydroxy, or acyloxy; R⁵ represents alkyl; R⁶ represents hydrogen.

2. The compound or the pharmaceutically acceptable salt thereof according to claim 1 wherein R¹ represents hydroxy; R² represents amino; R³ represents alkyl, hydroxy, mercapto or -S-X (wherein X represents alkyl which is either unsubstituted or substituted by a group selected from among hydroxy, carboxy, and alkoxyalkoxy); R⁴ represents hydrogen, hydroxy, acyloxy, or alkoxy; R⁵ represents alkyl or aryl; R⁶ represents hydrogen.

3. The compound or the pharmaceutically acceptable salt thereof according to claim 1 wherein R¹ represents amino or -NR¹¹R¹² (wherein R¹¹ and R¹² may be the same or different and each represents alkyl); R² represents hydrogen; R³ represents hydrogen or halogen; R⁴ represents hydrogen, hydroxy, alkoxy or -OSO₂-Y (wherein Y represents aryl which is either unsubstituted or substituted by alkyl or alkyl which is either unsubstituted or substituted by halogen); R⁵ represents alkyl; R⁶ represents hydrogen, halogen or alkyl; R⁴ and R⁶ may jointly represent =CH₂; R³ and R⁶ may jointly represent -S-; in the case that R⁴ and R⁶ are independent of each other, either one represents hydrogen.

4. The compound or the pharmaceutically acceptable salt thereof according to claim 1 wherein R¹ represents amino; R² represents hydrogen; R³ represents hydroxy, mercapto or -S-X (wherein X represents alkyl which is either unsubstituted or substituted by hydroxy or alkoxyalkoxy), cycloalkyl, or aryl which is either unsubstituted or substituted by halogen); R⁴ represents hydrogen, hydroxy or alkoxy; R⁵ represents alkyl; R⁶ represents hydrogen.

5. A pharmaceutical composition which comprises the compound or the pharmaceutically acceptable salt thereof according to claim 1 as an active ingredient.

6. An antidementia drug, an antiarrhythmic, a therapeutic drug for bronchial asthma, an antiinflammatory agent, a platelet aggregation inhibitor, an antihypertensive, a diuretic, or a muscle relaxant, which comprises the compound or the pharmaceutically acceptable salt thereof according to claim 1 as an active ingredient.

7. A pharmaceutical composition which comprises the compound or the pharmaceutically acceptable salt thereof according to claim 2 as an active ingredient.

8. An antidementia drug, an antiarrhythmic, a therapeutic drug for bronchial asthma, an antiinflammatory agent, a platelet aggregation inhibitor, an antihypertensive, a diuretic, or a muscle relaxant, which comprises the compound or the pharmaceutically acceptable salt thereof according to claim 2 as an active ingredient.

9. A pharmaceutical composition which comprises the compound or the pharmaceutically acceptable salt thereof according to claim 3 as an active ingredient.

10. An antidementia drug, an antiarrhythmic, a therapeutic drug for bronchial asthma, an antiinflammatory agent, a platelet aggregation inhibitor, an antihypertensive, a diuretic, or a muscle relaxant, which comprises the compound or the pharmaceutically acceptable salt thereof according to claim 3 as an active ingredient.

11. A pharmaceutical composition which comprises the compound or the pharmaceutically acceptable salt thereof according to claim 4 as an active ingredient.

12. An antidementia drug, an antiarrhythmic, a therapeutic drug for bronchial asthma, an antiinflammatory agent, a platelet aggregation inhibitor, an antihypertensive, a diuretic, or a muscle relaxant, which comprises the compound or the pharmaceutically acceptable salt thereof according to claim 4 as an active ingredient.
